# EUROPÄISCHE PATENTSCHRIFT

(11) **EP 4 256 306 B1**
(45) Veröffentlichungstag und Bekanntmachung des Hinweises auf die Patenterteilung: **26.02.2025**
(21) Anmeldenummer: 21836317.4
(22) Anmeldetag: 03.12.2021
(51) Int. Cl.: G01N 21/25, G01N 21/27, G01N 21/29, G01N 21/78, G01N 33/18, G01N 35/10, G01N 21/11, G01N 21/41, G01N 21/80, G01N 21/17, G01N 35/04

(54) **PIPETTIERROBOTER ZUR BESTIMMUNG ZUMINDEST EINER WASSEREIGENSCHAFT**
PIPETTING ROBOT FOR DETERMINING AT LEAST ONE WATER CHARACTERISTIC
ROBOT DE PIPETTAGE POUR LA DÉTERMINATION D'AU MOINS UNE CARACTÉRISTIQUE DE L'EAU

(30) Priorität: 04.12.2020 AT 603602020
(43) Veröffentlichungstag der Anmeldung: 11.10.2023
(73) Patentinhaber: Kofler GmbH, 7540 Güssing (AT)
(72) Erfinder: KOFLER, Stefan, 8010 Graz (AT)
(74) Vertreter: Schwarz & Partner Patentanwälte GmbH
(86) Internationale Anmeldenummer: PCT/AT2021/060461
(87) Internationale Veröffentlichungsnummer: WO 2022/115895

(56) Entgegenhaltungen:
- EP-A1- 3 566 573
- WO-A1-2013/104365
- US-B1- 9 689 803
- COSTA DIOGO ET AL: "The Nutrient App: Developing a smartphone application for on-site instantaneous community-based NO3 and PO4 monitoring", ENVIRONMENTAL MODELLING & SOFTWARE, ELSEVIER, AMSTERDAM, NL, vol. 133, 29 August 2020 (2020-08-29), XP086284567, ISSN: 1364-8152, [retrieved on 20200829], DOI: 10.1016/J.ENVSOFT.2020.104829

## Beschreibung

Die Erfindung betrifft einen Pipettierroboter zur Bestimmung zumindest einer vorbestimmten Eigenschaft von Probewasser, insbesondere von Aquarienwasser. In einem weiteren Aspekt betrifft die Erfindung eine Verwendung des Pipettierroboters und ein Verfahren zur Bestimmung der zumindest einen vorbestimmten Eigenschaft mittels des Pipettierroboters.

Aus der Aquarientechnik ist bekannt, dass die Wasserqualität in Aquarien die Lebensdauer der in den Aquarien gehaltenen Lebewesen stark beeinflusst. Aquarieninhaber sind daher bemüht, die Wasserqualität im Aquarium konstant zu halten und in regelmäßigen Abständen zu warten.

Um die Qualität vom Wasser zu bewerten, werden regelmäßig die wichtigsten Wasserwerte gemessen. Dies erfolgt meistens über sogenannte Tropfentests. Das Messen der Wasserwerte dauert bei einem durchschnittlichen Aquarium etwa 30 bis 45 Minuten und ist fehleranfällig, wenn es manuell durchgeführt wird. Es ist daher eine generelle Bestrebung, die Wasserwerte automatisiert und regelmäßig zu überprüfen.

Aus dem Stand der Technik ist die KR 20200064967 A bekannt, die ein System zur Messung von Wassereigenschaften eines Aquariums offenbart. Es können Reagenzien mit Probewasser vermischt werden, wobei der Farbumschlag mittels eines Kolorimeters ermittelt wird.

EP 3 566 573 A1 beschreibt ein automatisches System zur Einstellung der Wasserqualität von Aquarienwasser und ein automatisches Verfahren zur Einstellung der Wasserqualität, wobei das System einen Reaktionsbehälter, eine Probentropfvorrichtung, eine Titranstropfvorrichtung, einen Farbsensor, eine Lichtschranke, eine Abfallbeseitigungsvorrichtung, eine Steuereinheit und eine Zusatzvorrichtung umfasst, und wobei das System zur Einstellung der Wasserqualität den Gehalt eines bestimmten Elements in einer wässrigen Lösung durch Titration quantifiziert und zur Einstellung der Wasserqualität einen Zusatzstoff in die wässrige Lösung gibt, um den Gehalt des spezifischen Elements in der wässrigen Lösung zu stabilisieren.

WO 2013/104365 A1 beschreibt eine automatische Maschine zur Überprüfung von Behältern, bei der verschiedene grafische Muster zum Testen verschiedener optischer Eigenschaften einer Prüfstation, wie z.B. Fokus, Kontrast, Ausrichtung, etc. verwendet werden, wobei durch unterschiedliche Einfärbung von Linien, Flächen und farbigen Feldern, eventuell in Kombination mit Graustufenfeldern, wie sie z.B. von Fernsehtestkarten bekannt sind, die Farberfassung und -auswertung der Prüfstationen getestet werden kann. Die Prüfstationen verwenden Farbsensoren, Farbkameras und/ oder Bewertungsalgorithmen, die Farben berücksichtigen.

Aus abliegenden Gebieten, die nicht unmittelbar für die Aquarientechnik spezialisiert sind, sind die US 2007178010 A1, WO 2006037941 A1, US 2005276724 A1, US 2005 276724 A1, CN 109839490 A, DE 202008006338 U1, DE102008050092 A1 und die JP2000121411 A bekannt. Diese Dokumente zeigen jeweils, dass entweder eine Farbe oder ein Füllstand von Wasser mittels eines Kolorimeters bzw. einer Kamera gemessen werden kann. Keines dieser Systeme eignet sich jedoch unmittelbar für das Anwendungsgebiet der Aquarientechnik, wo möglichst alle Funktionen für den Endbenutzer automatisiert werden sollen.

Die vorliegende Erfindung ist in Anspruch 1 definiert.

Es ist die Aufgabe der Erfindung, ein vollautomatisiertes Verfahren bzw. einen zugehörigen Pipettierroboter zur Bestimmung zumindest einer vorbestimmten Eigenschaft von Probewasser, insbesondere Aquarienwasser, zu schaffen, das bzw. der die vorbestimmte Eigenschaft besonders genau und fehlerunanfällig bestimmten kann.

Diese Aufgabe wird erfindungsgemäß gelöst durch einen Pipettierroboter zur Bestimmung zumindest einer vorbestimmten Eigenschaft von Probewasser, insbesondere von Aquarienwasser, umfassend
- eine Messküvette zur Aufnahme des Probewassers,
- zumindest einen Reagenzbehälter zur Aufnahme eines flüssigen Reagenzes, mittels welches Reagenzes ein für die vorbestimmte Eigenschaft repräsentativer Farbumschlag im Probewasser herbeiführbar ist,
- eine Pumpe zum Verbringen des Reagenzes vom Reagenzbehälter in die Messküvette über einen Reagenzeinlass an einer Oberseite der Messküvette, wodurch das Reagenz in die Messküvette eintropfbar ist,
- eine Kamera zur Aufnahme von Bildern der Messküvette und des in der Messküvette befindlichen Probewassers,
- einen Hintergrund, welcher auf der der Kamera abgewandten Seite der Messküvette angeordnet ist und im Blickfeld der Kamera liegt, wobei auf dem Hintergrund zur Kontrastgebung Flächen in zumindest zwei unterschiedlichen Farben in einem vorbestimmten Muster abgebildet sind, wobei das Muster bevorzugt ein Schachbrettmuster mit schwarzen und weißen Flächen ist, und wobei auf dem Hintergrund zur Farbreferenzgebung zumindest drei Referenzfarbpunkte abgebildet sind, von denen zumindest einer Rot, einer Grün und einer Blau ist, und
- eine Auswerteeinheit, die dazu ausgebildet ist, anhand der von der Kamera aufgezeichneten Bilder die Füllmenge des Probewassers in der Messküvette zu bestimmen, die Anzahl von in die Messküvette eingetropften Reagenztröpfchen zu zählen und aus der gezählten Anzahl an Reagenztröpfchen in Kombination mit einem aus den Bildern ermittelten Farbumschlag des Probewassers die zumindest eine vorbestimmte Eigenschaft des Probewassers zu bestimmen.

Der erfindungsgemäße Pipettierroboter ermöglicht eine vollautomatisierte Analyse von Probewasser und kann dadurch in einfacher Weise insbesondere mit einem Aquarium kombiniert werden. Der Pipettierroboter eignet sich aufgrund seiner Fehlerunanfälligkeit und Selbstständigkeit insbesondere für den Heimgebrauch, sodass auch Benutzer ohne Laborkenntnis die Eigenschaften des Aquarienwassers bestimmen können.

Um diese vollautomatisierte Analyse durchzuführen, verbringt der Pipettierroboter automatisch eine vorbestimmte Menge von Aquariumwasser und ein oder mehreren Reagenzien in die Messküvette. Die Auswerteeinheit ermöglicht in Kombination mit der Kamera und dem erfindungsgemäßen Hintergrund, dass einerseits Reagenztröpfchen genau abzählbar sind und andererseits auch ein Farbumschlag des Probewassers genau bestimmbar ist. Eine subjektive Einschätzung des Farbumschlages durch den Benutzer entfällt daher vollständig.

Im erfindungsgemäßen Pipettierroboter trägt der Hintergrund zur besonders genauen Bestimmung des Farbumschlages bzw. zum korrekten Zählen der Tropfen bei. Das Muster ermöglicht, dass ein Tropfen im Bild deutlicher hervortritt, da sich durch die Lichtbrechung im Tropfen die Konturen des Musters im Bild bewegen. Die Referenzfarbpunkte ermöglichen einerseits das Kalibrieren der Position der Kamera, falls diese nicht genau eingebaut wurde, und andererseits können Farbtoleranzen der Kamera und von LEDs ausgeglichen werden, die die Messküvette beleuchten.

Im Gegensatz zum System der KR 20200064967 A wird die Kamera für mehr Zwecke als nur für die Bestimmung der Farbe eingesetzt, nämlich insbesondere auch zur Bestimmung des Wasserstandes und zum Zählen der Tröpfchen. Weiters kann durch den Einsatz des erfindungsgemäßen Hintergrunds auch die Farbe des Probewassers in der Messküvette wesentlich besser bestimmt werden.

In einer bevorzugten Ausführungsform ist der Reagenzeinlass dazu ausgebildet, Reagenztröpfchen mit einer vorbestimmten Tröpfchengröße auszugeben, wobei die genannten Flächen eine durchschnittliche Höhe und/oder eine durchschnittliche Länge haben, die maximal der Hälfte der Tröpfchengröße entspricht. Dadurch können die Reagenztröpfchen leichter durch die Auswerteeinheit erkannt werden, wodurch sich die Genauigkeit und Fehlerunanfälligkeit des Pipettierroboters weiter erhöht.

In einer ersten bevorzugten Ausführungsform ist die Pumpe dazu ausgebildet, einen Überdruck im Reagenzbehälter aufzubauen, um das Reagenz über einen in den Reagenzbehälter ragenden Schlauch zum Reagenzeinlass der Messküvette zu verbringen. Dies ermöglicht ein besonders effizientes Verbringen des Reagenzes in die Messküvette, da die Pumpe nicht unmittelbar mit dem Reagenzbehälter in Verbindung stehen muss. Diese Möglichkeit ist insbesondere dann vorteilhaft, wenn mehrere Reagenzbehälter im Pipettierroboter vorgesehen sind.

In einer zweiten bevorzugten Ausführungsform kann die Pumpe zumindest einen motorlosen Pumpenkopf und einen mit dem motorlosen Pumpenkopf koppelbaren Pumpenmotor umfassen, wobei der Pipettierroboter bevorzugt zumindest zwei Reagenzbehälter und einen eigenen motorlosen Pumpenkopf für jeden der zumindest zwei Reagenzbehälter umfasst, und wobei der Pumpenmotor auf einer Führung gelagert ist und durch Verschieben auf der Führung selektiv mit den Pumpenköpfen der zumindest zwei Reagenzbehälter koppelbar ist. In dieser Ausführungsform ist besonders vorteilhaft, dass der Pumpenmotor von den Pumpenköpfen koppelbar ist, wodurch beispielsweise vorgesehen werden kann, dass sich mehrere Pumpenköpfe einen einzigen Pumpenmotor teilen. Dadurch kann die Anzahl der Motoren im System reduziert werden, was den Pipettierroboter weniger fehleranfällig macht. Die Pumpenköpfe umfassen beispielswiese einen rotierbaren Exzenter und können als Verdrängerpumpen bzw. Peristaltikpumpen ausgebildet werden, jeweils ohne Motor und bevorzugt auch ohne Getriebe. Der Pumpenmotor, beispielsweise ein Schrittmotor, kann auf der genannten Führung, die in der Regel eine Linearführung ist, angeordnet werden und selektiv mit einem der Pumpenköpfe gekoppelt werden, indem er auf der Linearführung zum gewünschten Pumpenkopf verfährt.

In der genannten Ausführungsform umfasst der Pumpenmotor bevorzugt eine rotierbare Kopplungseinheit, welche zur Kopplung mit dem motorlosen Pumpenkopf in eine gegengleiche Aufnahme des motorlosen Pumpenkopfs einführbar ist, wobei die Aufnahme des Pumpenkopfs bevorzugt zumindest zwei symmetrisch um eine Rotationsachse angeordnete Stifte aufweist, die im Querschnitt die Form eines Dreiecks oder Kreissektors haben. Die rotierbare Kopplungseinheit hat sich in Versuchen als bevorzugte Schnittstelle zu den Pumpenköpfen bewährt. Im einfachsten Fall kann die Kopplungseinheit die Form eines Quaders haben, d.h. einen rechteckigen Querschnitt normal zur Rotationsachse aufweisen, und so in der Art eines Schlitzschraubenziehers die Aufnahme des Pumpenkopfes und dadurch beispielsweise deren Exzenter rotieren, sodass der Pumpenkopf Flüssigkeit fördert, wenn er mit dem Pumpenmotor verbunden ist. Es hat sich herausgestellt, dass eine quaderförmige Kopplungseinheit überaus genau positioniert werden muss, wenn die Aufnahme durch zwei gegengleich angeordnete Quader gebildet wird, zwischen die die Kopplungseinheit eingeführt wird. Wenn der Anstellwinkel nicht genau eingestellt wird, kann der Pumpenmotor leicht mit der Aufnahme der Pumpenköpfe kollidieren. Wenn die Aufnahme jedoch durch zwei Stifte gebildet wird, die im Querschnitt die Form eines Dreiecks oder Kreissektors haben, muss der Anstellwinkel weniger genau sein und Kollisionen treten weniger selten auf.

Es ist insbesondere von Vorteil, wenn der Pipettierroboter zumindest zwei, bevorzugt zumindest zehn, Reagenzbehälter aufweist, die jeweils mit der Pumpe und der Messküvette in Verbindung stehen, wobei die Reagenzbehälter jeweils ein ansteuerbares Ventil, bevorzugt ein Magnetventil, zur selektiven Verbindung zur Pumpe und/oder zur Messküvette umfassen. Dadurch können diverse Reagenzien im Pipettierroboter gelagert werden, um sequentiell verschiedene Eigenschaften des Probewassers zu bestimmen. Die Magnetventile ermöglichen hierbei, dass eine einzige Pumpe für mehrere Reagenzbehälter eingesetzt werden kann, wobei die Magnetventile individuell ansteuerbar sind.

Um ein Auswechseln bzw. Nachfüllen der Reagenzien zu ermöglichen, können der bzw. die Reagenzbehälter auswechselbar im Pipettierroboter vorgesehen sein, wobei der bzw. die Reagenzbehälter zur besonders einfachen Handhabung hängend in den Pipettierroboter einbringbar ist bzw. sind.

In der einfachsten Ausführungsform könnte manuell eine vorbestimmte Menge an Probeflüssigkeit in die Messküvette eingefüllt werden. Bevorzugt ist jedoch, wenn der Pipettierroboter mit einer Quelle des Probewassers verbunden und dazu ausgebildet ist, so lange Probewasser in die Messküvette zu pumpen, bis die Auswerteeinheit anhand der von der Kamera aufgenommenen Bilder eine gewünschte Füllmenge in der Messküvette erkennt. Dadurch kann der Pipettierroboter Messvorgänge selbsttätig starten, ohne dass das Probewasser in einer vorab bestimmten Füllmenge manuell in die Messküvette verbracht werden müsste. Alternativ oder zusätzlich kann die Auswerteeinheit dazu verwendet werden, die Pumpen zu kalibrieren. So kann z.B. mittels Auswerteeinheit festgestellt werden, dass die Pumpe für das Dosieren von 10 ml Probewasser 12 Sekunden benötigt. Daraus ergibt sich die Zeitdauer für alle anderen Volumen, die dosiert werden können.

Die Erkennung der Füllmenge an Probewasser kann auf verschiedene Weisen erfolgen. Bevorzugt ist jedoch, wenn die Auswerteeinheit dazu ausgebildet ist, das Erreichen der gewünschten Füllmenge durch Abgleich mit einer in der Auswerteeinheit hinterlegten Referenzposition in den von der Kamera aufgenommenen Bildern zu bestimmen. Die Referenzposition kann beispielsweise dadurch in der Auswerteeinheit hinterlegt werden, dass zuvor manuell Probewasser mit der gewünschten Füllmenge in die Messküvette eingebracht wird und die Auswerteeinheit die Füllstandshöhe dieser Füllmenge als Referenzposition, z.B. Pixelwert, hinterlegt. Alternativ oder zusätzlich können auch Marker an der Messküvette oder dem Hintergrund aufgetragen sein, welche die Füllmenge anzeigen.

Wenn die Füllmenge mittels der Auswerteeinheit bestimmt wird, ist besonders bevorzugt, wenn die Messküvette einen transparenten Probewassereinlass zum Zuführen des Probewassers aufweist und der Pipettierroboter dazu ausgebildet ist, einen Pumpvorgang des Probewassers in die Messküvette so lange mit einer ersten Probewasserpumpgeschwindigkeit durchzuführen, bis die Auswerteeinheit das Vorhandensein des Probewassers im Probewassereinlass detektiert, und den Pumpvorgang so lange mit einer zweiten Probewasserpumpgeschwindigkeit durchzuführen, die geringer ist als die erste Probewasserpumpgeschwindigkeit, bis die Auswerteeinheit Probewasser an der Referenzposition erkennt. Dies ist bevorzugt, da der Zuführschlauch des Probewassers entleert sein kann und sich daher Luft im Zuführschlauch befindet. Erfindungsgemäß kann daher eingangs schneller gepumpt werden, bis Probewasser tatsächlich in die Messküvette eingebracht wird. Nach Erreichen der gewünschten Füllmenge wird der Pumpvorgang beendet.

Auch für das Eintropfen des Reagenzes kann ein ähnlicher Vorgang durchgeführt werden. Hierbei kann der Reagenzeinlass transparent sein, wobei die Pumpe dazu ausgebildet ist, einen Pumpvorgang des Reagenzes so lange mit einer ersten Geschwindigkeit durchzuführen, bis die Auswerteeinheit das Vorhandensein des Reagenzes im Reagenzeinlass detektiert, und den Pumpvorgang so lange mit einer zweiten Geschwindigkeit durchzuführen, die geringer ist als die erste Geschwindigkeit, bis die Auswerteeinheit eine vorbestimmte Anzahl von Reagenztröpfchen gezählt oder einen Farbumschlag im Probewasser detektiert hat.

Es versteht sich, dass hierbei bevorzugt wird, wenn die Auswerteeinheit dazu ausgebildet ist, die vom Reagenzeinlass in die Messküvette tropfenden Reagenztröpfchen zu zählen und die Pumpe abzuschalten, sobald eine vorbestimmte Anzahl von Reagenztröpfchen oder ein vorbestimmter Farbumschlag des Probewassers erkannt wurde.

Der Pipettierroboter kann jedoch auch dazu ausgebildet sein, Eigenschaften des Probewassers zu bestimmen, die nicht mittels eines Reagenzes bestimmt werden. Zur Bestimmung des Salzgehaltes kann der Hintergrund oder die Messküvette beispielsweise einen vertikalen Streifen, bevorzugt einen vertikalen roten Streifen, zur Ermittlung eines Salzgehaltes aufweisen.

In dieser Ausführungsform kann die Messküvette eine Lupe aufweisen, wobei die Kamera, die Lupe und der vertikale Streifen derart positioniert sind, dass der vertikale Streifen auf den von der Kamera aufgenommenen Bildern bei einem vorbestimmten Salzgehalt, bevorzugt 3,5 %, im Probewasser vergrößert dargestellt ist. Dadurch kann der besonders bevorzugte Messbereich noch genauer analysiert werden.

Weiters kann der Hintergrund oder die Messküvette zumindest einen horizontalen Streifen, bevorzugt zumindest einen horizontalen grünen Streifen, zur Plausibilisierung einer Füllmenge aufweisen. Dadurch wird die Fehleranfälligkeit des Pipettierroboters beim Einfüllen der Messflüssigkeit weiter reduziert.

Damit das in der Messküvette befindliche Probewasser nicht manuell entleert werden muss, kann vorgesehen werden, dass die Messküvette an der Unterseite eine Auslassöffnung zum Entleeren der Messküvette aufweist. Dadurch können mehrere Messungen am Probewasser vollautomatisch sequentiell durchgeführt werden.

In der letztgenannten Ausführungsform ist bevorzugt, dass der Pipettierroboter dazu ausgebildet ist, zum Mischen des Probewassers und des eingetropften Reagenzes zumindest eine Luftblase über die genannte Auslassöffnung einströmen zu lassen. Dadurch kann ein schnelles Mischen des Probewassers mit dem Reagenz erzielt werden, ohne dass es weiterer Hilfsmittel oder einer Vibration bzw. eines Schüttelns der Messküvette bedarf.

Mit den oben beschriebenen Reagenzien kann beispielsweise der pH-Wert des Probewassers ermittelt werden. Um den pH-Wert jedoch ständig zu messen, d.h. auch während der Bestimmung von anderen Eigenschaften des Probewassers mittels Reagenzien, kann der Pipettierroboter mit einer eigenen Sonde zur Messung des pH-Wertes verbunden werden. Eine Kalibrierung dieser Sonde kann erzielt werden, indem die vorbestimmte Eigenschaft ein pH-Wert ist, wobei das Reagenz derart gewählt ist, einen Farbumschlag des Probewassers in Abhängigkeit des pH-Wertes zu bewirken, wobei die Auswerteeinheit dazu ausgebildet ist, in Abhängigkeit des Farbumschlages den pH-Wert des Probewassers zu bestimmen, wobei weiters eine Sonde zur Messung des pH-Wertes in die Messküvette eingeführt und die Auswerteeinheit dazu ausgebildet ist, die Sonde mittels des pH-Wertes zu kalibrieren, der von der Auswerteeinheit über den Farbumschlag bestimmt wurde.

Die korrekte Bestimmung des Farbumschlages ist nicht nur von der Kamera bzw. der Kalibrierung der Kamera abhängig, sondern auch von dem die Messküvette beleuchtenden Licht. Bevorzugt umfasst der Pipettierroboter daher eine lichtundurchlässige Kammer, in welcher sich die Messküvette befindet, wobei zumindest eine Leuchte, bevorzugt eine LED-Leuchte, zur Ausleuchtung der Messküvette in der Kammer angeordnet ist, und wobei sich die Leuchte bevorzugt neben der Messküvette befindet. Eine weitere Leuchte kann sich gegebenenfalls über oder unter der Messküvette befinden. Dadurch kann der Farbumschlag bzw. die Füllstandshöhe des Probewassers besonders genau bestimmt werden.

Um Fehlmessungen der vorbestimmten Eigenschaft zu verhindern, kann der Pipettierroboter einen Beschleunigungsmesssensor und/oder einen Neigungssensor umfassen, wobei die Auswerteeinheit dazu ausgebildet ist, eine Messung der vorbestimmten Eigenschaft zu invalideren, wenn der Beschleunigungsmesssensor und/oder der Neigungssensor eine Schieflage oder einen Stoß des Pipettierroboters anzeigt.

Besonders bevorzugt umfasst der Pipettierroboter ein Gefäß, in welchem ein Pulver gelagert ist, mittels welchem Pulver ein für die vorbestimmte Eigenschaft repräsentativer Farbumschlag im Probewasser herbeiführbar ist, wobei das Gefäß zumindest auf einer ersten Seite, bevorzugt auf der Oberseite, mittels einer ersten Folie verschlossen ist, wobei der Pipettierroboter bevorzugt ferner eine Nadel umfasst und dazu ausgebildet ist, die Folie mittels der Nadel einzustechen, eine Flüssigkeit zur Lösung des Pulvers in das Gefäß zu füllen und das in der Flüssigkeit gelöste Pulver in die Messküvette zu überführen, wobei das Gefäß bevorzugt auch an einer zweiten Seite, bevorzugt auf der Unterseite, mittels einer zweiten Folie verschlossen ist, durch welche das in der Flüssigkeit gelöste Pulver in die Messküvette überführbar ist. Dadurch wird eine einfache Möglichkeit geschaffen, nicht nur flüssige Reagenzien zur Bestimmung der Wassereigenschaften einzusetzen, sondern auch pulverförmige Reagenzien. Die Folie ermöglicht, dass das Pulver einerseits lange trocken gelagert werden kann und andererseits, dass das Pulver leicht freigegeben werden kann. Die Nadel ermöglicht ein einfaches Öffnen der Folie, sodass diese nicht abgezogen werden muss. Das Auflösen des Pulvers vor dem Überführen in die Messküvette hat den Vorteil, dass mittels der genannten Kamera genau überprüft werden kann, dass das Pulver überführt wurde, und dass kein Pulver im Gefäß verbleibt. Die zweite Folie hat den Vorteil, dass das Gefäß nicht verkippt werden muss, um das gelöste Pulver in die Messküvette zu überführen.

In der vorgenannten Ausführungsform ist weiters bevorzugt, wenn der Pipettierroboter einen bevorzugt modular in den Pipettierroboter einsetzbaren Kunststoffring umfassend zumindest zwei der genannten Gefäße, in denen jeweils Pulver gelagert ist, umfasst, wobei die Gefäße um den Umfang des Kunststoffrings verteilt angeordnet sind und der Pipettierroboter einen Steppmotor zum Drehen des Kunststoffringes umfasst, mittels welchem die Gefäße selektiv zur Nadel, zur Zuführstelle der Flüssigkeit und/oder zur Überführungsstelle zur Messküvette verdrehbar sind. Dadurch wird eine Möglichkeit geschaffen, mehrere Dosierungen von Pulver gleichzeitig aber getrennt voneinander bereitzustellen, um mehrere Messvorgänge durchzuführen ohne manuell Gefäße wechseln zu müssen. Nachdem alle Gefäße leer sind, kann der gesamte Kunststoffring ersetzt werden. Gegebenenfalls wird die Flüssigkeit zum Lösen des Pulvers unmittelbar über der Messküvette hinzugefügt, d.h. die zweite Stelle und die dritte Stelle können zusammenfallen, was insbesondere von Vorteil ist, wenn die zweite Folie wasserlöslich ist.

In einem zweiten Aspekt betrifft die Erfindung eine Verwendung des Pipettierroboters nach einer der vorgenannten Ausführungsformen zur Bestimmung einer vorbestimmten Eigenschaft von Probewasser, wobei das Probewasser Aquarienwasser ist und die vorbestimmte Eigenschaft bevorzugt eine Alkalinität, eine Calciumhärte, eine Magnesiumhärte, ein Phosphatwert, ein Nitratwert und/oder ein pH-Wert ist.

In einem dritten Aspekt betrifft die Erfindung ein Verfahren zum Bestimmen einer vorbestimmten Eigenschaft von Probewasser mittels eines Pipettierroboters nach einer der vorgenannten Ausführungsformen, umfassend die Schritte:
- Einfüllen von Probewasser in die Messküvette,
- Verbringen des Reagenzes vom Reagenzbehälter in die Messküvette mittels der Pumpe,
- Aufnehmen von Bildern der Messküvette durch die Kamera,
- Zählen von Reagenztröpfchen, die über den Reagenzeinlass in die Messküvette eingetropft werden, und Ermitteln eines Farbumschlages des Probewassers durch die Auswerteeinheit anhand der von der Kamera aufgenommenen Bilder, und
- Bestimmen der vorbestimmten Eigenschaft anhand der Anzahl der gezählten Reagenztröpfchen und/oder des ermittelten Farbumschlages. Beispielsweise kann ein Test auch eine bestimmte Anzahl von Reagenzien benötigen und aus der Verfärbung des Probewassers wird der entsprechende Wasserwert ermittelt. Bei einer Phosphat Messung kann beispielsweise gelten: je dunkel-blauer sich das Probewasser verfärbt, desto höher ist der Phosphatwert.

Die Verwendung und das Verfahren weisen dieselben Vorteile auf und können mit denselben Modifikationen durchgeführt werden, wie sie für den Pipettierroboter erläutert wurden.

Vorteilhafte und nicht einschränkende Ausführungsformen der Erfindung werden nachfolgend anhand der Zeichnungen näher erläutert.
Figur 1 zeigt eine beispielhafte Anordnung von Reagenzbehältern und der Messküvette des erfindungsgemäßen Pipettierroboters in einer schematischen Perspektivansicht.
Figur 2 zeigt die Anordnung der Kamera, der Messküvette und des Hintergrunds des erfindungsgemäßen Pipettierroboters in einer schematischen Perspektivansicht.
Figur 3 zeigt eine beispielhafte Ausführungsvariante des Hintergrundes des erfindungsgemäßen Pipettierroboters.

Die Figuren 4 und 5 zeigen bevorzugte Ausführungsformen des Pumpsystems des erfindungsgemäßen Pipettierroboters.

Figur 6 zeigt eine Ausführungsform zur Überführung eines Pulvers in die Messküvette.

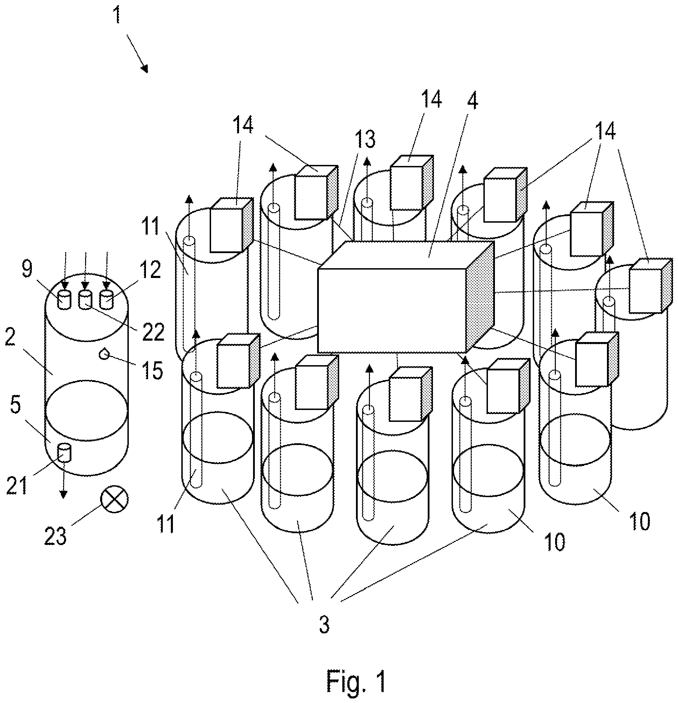

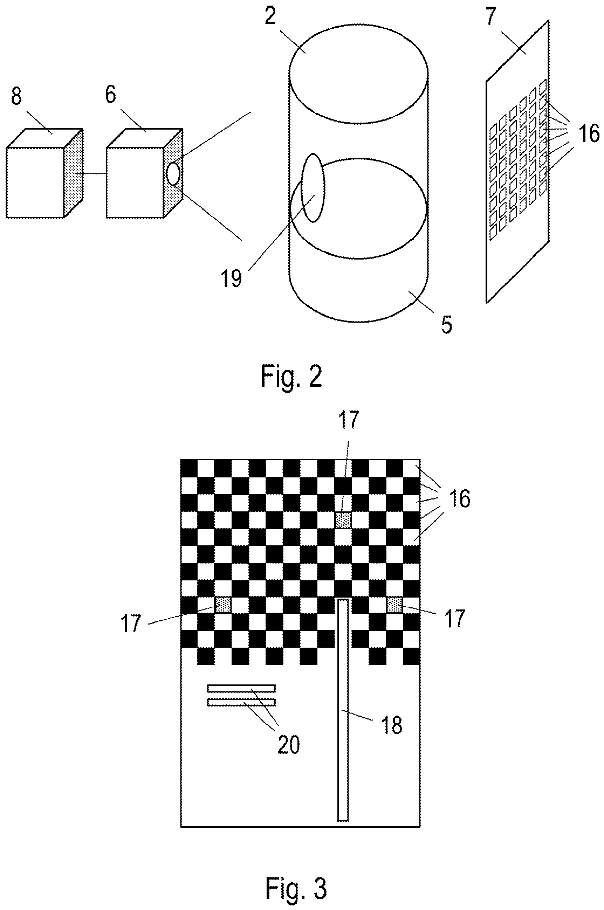

Figur 1 zeigt einen Pipettierroboter 1 mit einer Messküvette 2, elf Reagenzbehältern 3 und einer Pumpe 4. Der Pipettierroboter 1 ist dazu ausgebildet, zumindest eine vorbestimmte Eigenschaft von Probewasser 5 zu bestimmen, das einem nicht weiter dargestellten Aquarium entnommen und der Messküvette 2 zugeführt wurde. Um die vorbestimmte Eigenschaft zu bestimmen, weist der Pipettierroboter 1 ferner die in Figur 2 dargestellte Kamera 6, den Hintergrund 7 und die Auswerteeinheit 8 auf. Für die unten beschriebenen Zwecke nimmt die Kamera 6 bevorzugt Bilder mit einer Rate von 90 fps (frames per second) auf. Die Auswerteeinheit 8 kann ein einfacher Prozessor, ein Einplatinencomputer wie ein RaspberryPi oder eine sonstige Recheneinheit sein. Zu den unten beschriebenen Zwecken kann die Auswerteeinheit 8 die Pumpe und/oder die Kamera 6 ansteuern.

Die vorbestimmte Eigenschaft kann beispielsweise ein pH-Wert oder eine Alkalinität sein. Sobald der Pipettierroboter 1 die vorbestimmte Eigenschaft bestimmt hat, kann diese auf einer Anzeige, gegebenenfalls bei Überschreitung oder Unterschreitung eines Schwellwerts mit Warnlicht, angezeigt werden oder einer Steuereinrichtung zuführt, die das Aquarium in Abhängigkeit der gemessenen Eigenschaft wartet, beispielsweise durch Zugeben von Zusatzstoffen, z.B. Salz oder Regulatoren für den Calcium-Wertes.

Es sei jedoch darauf hingewiesen, dass der hierin beschriebene Pipettierroboter 1 nicht auf die Anwendung der Messung von Aquarienwasser beschränkt ist, sondern auch zur Messung von Meerwasser oder Poolwasser eingesetzt werden kann. Bevorzugt ist jedoch die Messung von Eigenschaften des Wassers von Salzwasseraquarien oder Süßwasseraquarien.

Die Messküvette 2 hat ein Volumen, das jedenfalls größer ist als das Messvolumen an Probewasser 5, das zur Bestimmung der vorbestimmten Eigenschaft benötigt wird, welches Messvolumen in der Regel 2 ml, 5 ml, 10 ml oder 15 ml beträgt. Die Messküvette 2 ist zumindest teilweise transparent ausgebildet, sodass die Kamera 6 Bilder der in der Messküvette 2 befindlichen Flüssigkeit aufnehmen kann. Die Form der Messküvette 2 ist bevorzugt zylindrisch oder rechteckig.

Um Probewasser 5 vom Aquarium in die Messküvette 2 zu verbringen, kann ein nicht weiter dargestellter Schlauch vom Aquarium an einen Probewassereinlass 9 geführt sein. Der Probewassereinlass 9 befindet sich beispielsweise an einer Oberseite der Messküvette 2, sodass das Probewasser 5 in die Messküvette 2 fließen oder tropfen kann. Zum Verbringen des Probewassers 5 aus dem Aquarium in die Messküvette 2 kann die genannte Pumpe 4 oder eine weitere Pumpe eingesetzt werden.

Um zu bestimmen, wie viel Probewasser 5 sich in der Messküvette 2 befindet, kann initial eine vorbestimmte Füllmenge an Wasser, insbesondere Probewasser 5, in die Messküvette 2 eingefüllt werden, um zu bestimmen, welche Füllstandshöhe welcher Füllmenge entspricht. Üblicherweise werden nur solche definierte Füllmengen aufgezeichnet, die zur Bestimmung der vorbestimmten Eigenschaft benötigt werden, d.h. 2 ml, 5 ml, 10 ml und/oder 15 ml. Nachdem eine definierte Füllmenge in die Messküvette 2 eingefüllt wurde, nimmt die Kamera 6 ein Bild von der befüllten Messküvette 2 auf, das von der Auswerteeinheit 8 ausgewertet wird. Die Auswerteeinheit 8 zeichnet danach die Position im Bild auf, an dem die Füllstandshöhe ersichtlich ist und speichert diese als Referenzposition zu der Füllmenge ab, z.B. als "2 ml entspricht x = 234 Pixel, y = 123 Pixel". Soll nun eine Probeflüssigkeit 5 in die Messküvette 2 eingefüllt werden, wird im einfachsten Fall solange Probeflüssigkeit 5 gepumpt, bis die Auswerteeinheit 8 an dem die Probeflüssigkeit die vorab gespeicherte Referenzposition bzw. Füllstandshöhe im Bild erreicht.

In einem erweiterten Verfahren kann vorgesehen werden, dass eingangs solange Probeflüssigkeit 5 gepumpt wird, bis die Auswerteeinheit 8 im Probeflüssigkeit 5 Probewassereinlass 9 erkennt, der hierfür transparent ausgebildet ist. Dies wird deshalb vorgesehen, da Probewasser 5 in der Regel nicht sofort in die Messküvette 2 einzufließen beginnt, da der Schlauch nach dem Einfüllen bzw. Messen üblicherweise entleert wird. Dies kann beispielsweise derart technisch umgesetzt werden, dass in OpenCV eine "Region of Interest" im Bild über den Probewassereinlass 9 gesetzt wird. Sobald im Probewassereinlass 9 Probewasser 5 erkannt wird, kann die Pumpgeschwindigkeit verringert werden und es wird solange Probeflüssigkeit 5 weitergepumpt, bis die Auswerteeinheit 8 Probeflüssigkeit 5 an der vorab gespeicherten Referenzposition bzw. Füllstandshöhe im Bild erkennt. Hierfür kann in OpenCV beispielsweise die "Region of Interest" auf den Bereich der Referenzposition bzw. gewünschten Füllstandshöhe gesetzt werden. Die Erfindung ist jedoch nicht auf diese Einfüllverfahren des Probewassers 5 in die Messküvette 2 beschränkt, sondern könnte auch auf andere Art und Weise erfolgen.

Nachdem eine bestimmte Menge an Probewasser 5 in die Messküvette 2 eingefüllt wurde, wird ein Reagenz 10 aus einem der Reagenzbehälter 3 in die Messküvette 2 verbracht, um aus einem daraus resultierenden Farbumschlag mittels der Kamera 6 und der Auswerteeinheit 8 die vorbestimmte Eigenschaft zu bestimmen. Die Reagenzien 10 sind im Handel frei erhältlich und können je nach zu messender Eigenschaft vom Fachmann gewählt werden.

In der Ausführungsform von Figur 1 sind elf Reagenzbehälter 3 vorgesehen, die jeweils unterschiedliche Reagenzien 10 umfassen. In allgemeineren Ausführungsformen kann auch nur ein Reagenzbehälter 3 oder mehr als ein Reagenzbehälter 3 vorgesehen werden. Die Reagenzbehälter 3 werden bevorzugt auswechselbar im Pipettierroboter 1 vorgesehen, um einen Reagenzbehälter 3 auszutauschen, wenn sich kein Reagenz 10 mehr darin befindet. Bevorzugt werden die Reagenzbehälter 3 oben offen ausgestaltet, sodass diese in einem Transportzustand mit einer Kappe verschlossen werden können und für den Betriebszustand hängend in den Pipettierroboter 1 eingebracht, z.B. eingeschraubt, werden können.

Wie in Figur 1 dargestellt ragt ein Schlauch 11 in den Reagenzbehälter 3, wenn dieser in den Pipettierroboter 1 eingebracht ist. Der Schlauch 11 ragt fast bis ganz zum Boden des Reagenzbehälters 3, damit eine maximale Menge an Reagenz 10 aus diesem entnommen werden kann. Der Schlauch 11 verläuft folglich vom Reagenzbehälter 3 zu einem Reagenzeinlass 12 der Messküvette 2. Der Reagenzeinlass 12 ist an der Oberseite der Messküvette 2 vorgesehen, damit das Reagenz 10 in das Probewasser 5 tropfen kann. Bevorzugt ist an der Oberfläche der Messküvette ein Reagenzeinlass 12 für jeden Reagenzbehälter 3 vorgesehen, sowie der Probewassereinlass 9, ein unten näher beschriebener Osmosewassereinlass 22 und ein weiterer Einlass für eine Referenzlösung. Weist der Pipettierroboter 1 somit zehn bis zwölf Reagenzbehälter 3 auf, werden an der Messküvette 2 dreizehn bis fünfzehn Einlässe vorgesehen. In anderen Ausführungsformen könnten auch Einlässe zusammengelegt werden.

Um Reagenz 10 aus dem Reagenzbehälter 3 in die Messküvette 2 zu pumpen, ist die Pumpe 4 über eine Verbindung 13, beispielsweise einem weiteren Schlauch, mit dem Reagenzbehälter 3 verbunden, um darin einen Überdruck zu erzeugen. Durch den Überdruck strömt das Reagenz 10 vom Reagenzbehälter 3 über den Schlauch 11 in die Messküvette 2. Besonders ist hierbei insbesondere, dass die Pumpe 4 über Verbindungen 13 mit allen Reagenzbehältern 3 verbunden ist. Da in der Regel jedoch nur ein Reagenz 10 in die Messküvette 2 überführt werden soll, wird für jeden der Reagenzbehälter jeweils ein Magnetventil 14 vorgesehen, welches die Verbindung zwischen Pumpe 4 und Reagenzbehälter kappen bzw. öffnen kann. Die Magnetventile 14 können individuell angesteuert werden, sodass gezielt das gewünschte Reagenz 10 aus einem bestimmten Reagenzbehälter 3 in die Messküvette 2 verbracht werden kann.

Um den Pipettierroboter 1 möglichst kompakt auszubilden, können die Reagenzbehälter 3 wie dargestellt ringförmig um die Pumpe 4 angeordnet werden. In dieser Variante können die Komponenten des Pipettierroboters 1 besonders einfach von einem beispielsweise kuppelförmigen Gehäuse umschlossen werden. Es versteht sich jedoch, dass auch andere Ausgestaltungsvarianten möglich sind. Der erfindungsgemäße Pipettierroboter 1 kann besonders klein ausgebildet werden, beispielsweise kuppelförmig mit einem Durchmesser von ca. 25 cm und einer Höhe von 20 cm. In anderen Ausführungsvarianten kann der Pipettierroboter 1 auch würfelförmig mit einer maximalen Kantenlänge von 25 cm, 20 cm oder 15 cm ausgebildet sein. Der Pipettierroboter ist daher bevorzugt als tragbares Handgerät ausgebildet.

Wenn das Reagenz 10 am Reagenzeinlass 12 vorliegt, wird sich ein Reagenztröpfchen 15 von diesem lösen und durch die Messküvette 2 in das Probewasser 5 tropfen. Zur Bestimmung der vorbestimmten Eigenschaft ist das korrekte Zählen der Reagenztröpfchen 15 essentiell, da die gezählte Anzahl Aufschluss über die Eigenschaft gibt. Wenn beispielsweise die Alkalinität des Probewassers 5 bestimmt werden soll, wird das Reagenz 10 so lange in die Messküvette 2 getropft, bis ein Farbumschlag des Probewassers 5 ersichtlich ist. Die Anzahl der Reagenztröpfchen 15 gibt dadurch Aufschluss über die Alkalinität des Wassers. In einer anderen Messung, beispielsweise der Bestimmung des pH-Wertes, wird eine vorbestimmte Anzahl an Reagenztröpfchen 15 in das Probewasser 5 eingetropft und aus dem Farbwert des erreichten Farbumschlages wird die Eigenschaft bestimmt.

Um die Anzahl der in das Probewasser 5 tropfenden Reagenztröpfchen 15 zu bestimmen, werden die vorgenannte Kamera 6 und die Auswerteeinheit 8 eingesetzt. Die Kamera 6 nimmt während des Tropfvorganges Bilder auf und die Auswerteeinheit 8 ermittelt die Anzahl der in das Probewasser 5 getropften Reagenztröpfchen 15. Bevorzugt ist der Reagenzeinlass 12 um einen vorbestimmten Mindestabstand von der Füllstandshöhe des Probewassers 5 beabstandet, damit der Reagenztropfen 15 vollständig ersichtlich ist, bevor dieser in das Probewasser 5 eintropft. Der Mindestabstand kann beispielsweise zumindest 1 cm betragen.

In einer konkreten Umsetzung kann die Auswerteeinheit 8 zuerst erkennen, ob sich Reagenz 10 im Reagenzeinlass 12 befindet, der zu diesem Zweck transparent ausgebildet sein kann. Wird die Erkennung in OpenCV durchgeführt, kann die "Region of Interest" auf jenen Bereich gesetzt werden, an dem sich der transparente Reagenzeinlass 12 befindet. Danach ermittelt die Auswerteeinheit 8, ob sich ein Reagenztröpfchen 15 löst und durch die Messküvette 2 fällt, wodurch ein Zähler für die Anzahl an Reagenztröpfchen 15 um eins hinaufgesetzt werden kann. Wird die Erkennung in OpenCV durchgeführt, kann die "Region of Interest" beispielsweise auf einen Bereich gelegt werden, der sich 5 mm unterhalb des Reagenzeinlasses 12 befindet. Das Tropfen des Reagenztröpfchens 15 durch die "Region of Interest" kann über "Movement detection" festgestellt werden. Zur Tropfenerkennung beträgt die Bildrate der Kamera bevorzugt 90 fps.

Es hat sich nun jedoch herausgestellt, dass die Tropfenerkennung und die korrekte Erkennung des Farbumschlages nicht ohne weiteres durchführbar sind. Aus diesem Grund wird ein Hintergrund 7 vorgesehen, der in Figur 3 im Detail dargestellt ist. Der Hintergrund 7 ist bezüglich der Kamera 6 und der Messküvette 2 derart angeordnet, dass sich die Messküvette 2 zwischen der Kamera 6 und dem Hintergrund 7 befindet. Ein von der Kamera 6 aufgenommenes Bild umfasst somit eine Darstellung der Messküvette 2 und des Hintergrundes 7 hinter der zumindest teilweise transparenten Messküvette 2. Der Hintergrund 7 ist derart ausgebildet, dass er einerseits einen Kontrast für die Tropfenerkennung und andererseits Referenzfarben für die Erkennung des Farbumschlages bietet. Zur Kontrastgebung weist der Hintergrund 7 schwarze und weiße Flächen 16 in einem vorbestimmten Muster auf, beispielsweise einem Schachbrettmuster wie in Figur 3 dargestellt. In anderen Ausführungsformen kann das vorbestimmte Muster, wie in Figur 2 angedeutet, eine vollflächige weiße Farbe mit schwarzen Rechtecken sein, die in einem regelmäßigen Abstand zueinander angeordnet sind. Bevorzugt ist das vorbestimmte Muster ein regelmäßiges Muster, sodass beispielsweise auch ein Wabenmuster mit regelmäßig angeordneten Sechsecken eingesetzt werden kann. In wiederum anderen Ausführungsformen kann das Muster ein unregelmäßiges, mosaikartiges Muster sein. Bevorzugt wird weiters, dass schwarze Flächen nur an weiße Flächen angrenzen. Gegebenenfalls können einzelne der Flächen 16 auch in Grautönen oder anderen Farben ausgebildet werden.

Wenn das vorbestimmte Muster ein Schachbrettmuster ist, beträgt die Seitenlänge der rechteckigen Flächen 16 bevorzugt maximal der Hälfte der Breite bzw. des Durchmessers eines Reagenztröpfchens 15. Die Breite bzw. der Durchmesser des Reagenztröpfchens 15 ist in der Regel durch die Form des Reagenzeinlasses 12 bedingt. Im Allgemeinen, auch wenn das Muster kein Schachbrettmuster ist, beträgt die durchschnittliche Höhe bzw. durchschnittliche Breite der Flächen 16 bevorzugt maximal der Hälfte der Breite bzw. des Durchmessers eines Reagenztröpfchens 15. Das Muster ist zumindest in jenem Bereich des Hintergrundes 7 abgebildet, der sich im aufgenommenen Bild zwischen dem Reagenzeinlass 12 und der Probeflüssigkeit 5 befindet. Bevorzugt ist das Muster, d.h. die Form der Flächen und deren Farben, in der Auswerteeinheit 8 vorab hinterlegt.

Durch die im Muster angeordneten Flächen 16 sind abtropfende Reagenztröpfchen 15 besonders gut im von der Kamera 6 aufgenommenen Bild erkennbar, insbesondere wenn zumindest zwei Flächen 16 zumindest teilweise innerhalb der Abbildung eines Reagenztröpfchens 15 liegen, wodurch die Kontrastgebung des Hintergrunds 7 erzielt wird. Um die Referenzfarbgebung zu erzielen, sind auf dem Hintergrund überdies drei Referenzfarbpunkte 17 abgebildet, von denen zumindest einer Rot, einer Grün und einer Blau ist. Dadurch wird ermöglicht, dass die Auswerteeinheit 8 aus einem von der Kamera 6 aufgezeichneten Bild eine Referenz vor den gesamten Farbbereich ermitteln kann, der aus den drei Grundfarben Rot, Grün und Blau bestimmen lässt, wie dem Fachmann allgemein bekannt ist. Einerseits kann durch die Referenzfarbpunkte 17 die Position der Kamera 6 kalibriert werden, falls diese nicht genau eingebaut wurde, und andererseits können Farbtoleranzen der Kamera 6 und von unten näher beschriebenen LEDs ausgeglichen werden, die die Messküvette 2 beleuchten.

Im Allgemeinen können die Referenzfarbpunkte 17 auf einer beliebigen Position am Hintergrund 7 abgebildet sein, sofern diese im Bildbereich der Kamera 6 liegen. Die Referenzfarbpunkte 17 können eine beliebige Form aufweisen. Insbesondere können auch die Flächen des vorgenannten Musters als formgebende Elemente der Referenzfarbpunkte 17 dienen, wie dies im Beispiel der Figur 3 mit drei Referenzfarbpunkten 17 im Schachbrettmuster dargestellt ist. Bevorzugt sind die Position und der Farbwert der Referenzfarbpunkte 17 in der Auswerteeinheit 8 vorab hinterlegt.

Mit der vorgenannten Methode ist es möglich, eine vorbestimmte Eigenschaft des Probewassers 5 zu bestimmen, die durch einen Farbumschlag durch das Reagenz 10 ermittelbar ist. Andere Eigenschaften des Probewassers 5 können auch ohne Zugeben eines Reagenzes 10 ermittelt werden. Zur Bestimmung eines Salzgehaltes kann der Hintergrund 7 beispielsweise einen vertikalen Streifen 18 umfassen, der zur leichteren Erkennung durch die Auswerteeinheit 8 bevorzugt farbig, insbesondere rot, ausgestaltet ist und sich somit vom Muster abhebt. Der vertikale Streifen 18 ist derart am Hintergrund 7 abgebildet, dass er sich im Bild zumindest teilweise im Probewasser 5 befindet. In horizontaler Richtung ist der vertikale Streifen 18 bezogen auf die Messküvette 2 im von der Kamera aufgezeichneten Bild außermittig angeordnet. Durch die Lichtbrechung wird der vertikale Streifen 18 unterhalb der Wasseroberfläche versetzt dargestellt. Wasser hat die physikalische Eigenschaft, dass ein höherer Salzgehalt eine höhere optische Dichte und somit eine stärkere Lichtbrechung mit sich bringt, was zu einem höheren Versatz des vertikalen Streifens 18 führt. Dadurch kann der Salzgehalt des Probewassers 5 ermittelt werden.

Um den Salzgehalt noch genauer zu bestimmen, kann auf, vor oder hinter der Messküvette 2 eine Lupe 19 angebracht sein, um die Abweichung besser zu erkennen. Die Lupe 19 hat beispielsweise eine 10-fache Vergrößerung und wird derart positioniert, dass ein Salzgehalt um 3,5 % genauer erkennbar ist. Ist der Streifen 18 im von der Lupe 19 vergrößerten Bildabschnitt nicht erkennbar, kann darauf geschlossen werden, dass der Salzgehalt des Probewassers 5 zu stark von dem betrachteten Salzgehalt, in diesem Beispiel 3,5 %, abweicht, wodurch die Auswerteeinheit 8 eine entsprechende Meldung ausgeben kann.

In OpenCV kann die Funktion der Bestimmung der Salzgehaltserkennung beispielsweise wie folgt implementiert werden. Eingangs kann eine "Region of Interest" in einen Bereich gesetzt werden, der unterhalb der Wasseroberfläche und innerhalb der Messkette 2 liegt. Mittels "object find" kann der bevorzugt rote Strich 18 gesucht und anhand dessen Position der Salzgehalt bestimmt werden. Ist eine Lupe 19 vorhanden, kann in einem zweiten Schritt die "Region of Interest" in den Bereich der Lupe 19 gesetzt werden, um eine noch genauere Position des Streifens 18 zu bestimmen.

Zurückkommend auf die Bestimmung der Füllmenge kann der Hintergrund 7 auch hierfür ein darauf abgebildetes Element aufweisen, nämlich zumindest einen horizontalen Streifen 20, bevorzugt zumindest einen horizontalen grünen Streifen. Wenn sich der horizontale Streifen 20 unterhalb des Wasserspiegels befindet, wird dieser durch die Lichtbrechung verzerrt. Auch der horizontale Streifen 20 kann bezüglich der Messküvette 2 im von der Kamera aufgezeichneten Bild außermittig angeordnet sein. Üblicherweise dient der horizontale Streifen 20 der Plausibilisierung der Füllmenge, d.h. wird zusätzlich zu dem oben beschriebenen Verfahren eingesetzt. In anderen Ausführungsformen könnte das Verfahren zur Bestimmung der Füllmenge auch nur mit dem horizontalen Streifen 20 durchgeführt werden. Bevorzugt kann auch mehr als ein horizontaler Streifen 20 eingesetzt werden. Der oder die horizontalen Streifen befinden sich üblicherweise auf einer Höhe, die einer vorbestimmten Füllmenge entspricht, beispielsweise 2 ml, 5 ml, 10 ml oder 15 ml, und/oder knapp darunter, z.B. 0,1 ml darunter und/oder knapp darüber, z.B. 0,1 ml darüber.

Wie beschrieben können der vertikale Streifen 18 und der horizontale Streifen 20 auf dem Hintergrund 7 abgebildet sein. In anderen Ausführungsformen können diese Streifen 18, 20 jedoch auch unmittelbar auf der Messküvette 2 aufgedruckt sein. In der Regel ist der Hintergrund 7 eine ebene oder gekrümmte Bildfläche, beispielsweise bestehend aus Karton oder Kunststoff. Alternativ könnte der Hintergrund 7 unmittelbar auf die Messküvette 2 aufgedruckt sein. Der Hintergrund 7 ist bevorzugt undurchsichtig hinter dem gesamten Bereich der Messküvette 2 im ausgenommenen Bild. Die Streifen 18, 20 könnten auch auf einer zusätzlichen Bildfläche aufgebracht sein, die sich vor der Bildfläche mit dem Muster und den Referenzfarbpunkten 17 befindet.

Überdies können weitere Sensoren eingesetzt werden, um weitere Eigenschaften des Probewassers 5 zu bestimmen. Beispielsweise können eine Temperatursonde und/oder eine pH-Sonde vorgesehen werden, die jeweils mit dem Pipettierroboter 1 verbunden sind und sich jeweils an einem in das Aquarium eingeführten Schlauch befinden können, über den das Probewasser 5 in die Messküvette 2 verbracht wird. Mittels der pH-Sonde kann der pH-Wert auch ohne ein hierfür geeignetes Reagenz 10 gemessen werden kann. Somit kann beispielsweise eine Alkalinität über ein Reagenz 10 und gleichzeitig der pH-Wert über die pH-Sonde ermittelt werden. Die pH-Sonde sollte jedoch regelmäßig kalibriert werden, sodass der Pipettierroboter 1 den pH-Wert mittels eines Reagenzes 10 messen kann und anhand dieses Messwertes die pH-Sonde kalibriert.

An der Unterseite der Messküvette 2 kann sich ferner eine Auslassöffnung 21 befinden, durch die das in der Messküvette 2 befindliche Probewasser 5 aus der Messküvette 2 ausgelassen werden kann, um diese zu entleeren. Hierfür kann beispielsweise eine Dosierpumpe verwendet werden, die unterhalb der Messküvette 2 vorgesehen ist. Der Entleervorgang wird beispielsweise nach einem Messvorgang zur Bestimmung der vorgenannten Eigenschaft oder nach einer Fehlermeldung durchgeführt. Eine Fehlermeldung kann beispielsweise durch einen Beschleunigungsmesssensor und/oder der Neigungssensor nach Erkennung einer Schieflage oder eines Stoßes des Pipettierroboters 1 ausgegeben werden. Alternativ oder zusätzlich könnte eine derartige Fehlermeldung auch eine Messung invalidieren.

Nachdem das Probewasser 5 durch die Auslassöffnung 21 ausgelassen wurde, kann die Messküvette 2 mit osmotischem Wasser gespült werden, das über einen Osmosewassereinlass 22 an der Oberseite der Messküvette 2 in diese eingelassen wird. Hierdurch können Rückstände aus der Messküvette 2 beseitigt werden, wodurch auch für die nächste Messung gute Messwerte erzielbar sind. Auch hierfür kann die oben beschriebene Funktion des Erkennens des Wasserspiegels eingesetzt werden, um zu erkennen, ob die richtige Menge an Osmosewasser in die Messküvette 2 gepumpt wird.

Damit die Auswerteeinheit 8 Farbwerte genauer bestimmen kann, befindet sich die Messküvette 2 üblicherweise in einer lichtundurchlässigen Kammer, die mit zumindest einer Leuchte 23, bevorzugt zumindest einer LED-Leuchte, ausgeleuchtet wird. Damit kann eine homogene und konstante Ausleuchtung der Messküvette 2 erzielt werden. Um die Höhe des Wasserspiegels und somit den Füllstand in der Messküvette 2 leichter zu erkennen, wird die Leuchte 23 bevorzugt neben der Messküvette 2 angebracht. Alternativ oder zusätzlich kann die Leuchte 23 auch oberhalb oder unterhalb der Messküvette 2 angeordnet werden. Durch die Lichtbrechung an der Wasseroberfläche kommt diese dann klarer heraus.

Es ist überdies zu beachten, dass die Farbe nicht nur von der Farbe des mit dem Reagenz 10 vermengten Probewassers 5, sondern auch vom Farbspektrum der beleuchtenden Leuchte 23 abhängt. Es hat sich herausgestellt, dass es für die vorliegenden Zwecke effizienter bzw. kostengünstiger ist, zumindest zwei LED-Leuchten einzusetzen. Insbesondere kann eine erste LED-Leuchte eingesetzt werden, die Intensitätsspitzen im Bereich von 450 nm und 650 nm aufweist, und eine zweite LED-Leuchte, die eine Intensitätsspitze im Bereich von 500 nm aufweist. Diese LED-Leuchten 23 werden bevorzugt in einem 90°-Winkel zur Kamera 6 positioniert. Da es in der Messkammer zu Reflexionen kommen kann, kommt es vor, dass der Farbton des Probewassers 5 je nach Position im Bild anders aussieht. Zur Bestimmung des Farbwertes können daher Farbtöne gemittelt werden, die sich bevorzugt innerhalb einer "Region of Interest" befinden, wenn eine entsprechende Umsetzung in OpenCV erfolgt.

Wie bereits ausgeführt, werden die Reagenzien 10 von der Pumpe 4 in die Messküvette 2 verbracht. Das Probewasser 5 kann wiederum mit einer eigenen Pumpe vom Aquarium in die Messküvette verbracht werden. Mittels einer weiteren Pumpe kann Osmosewasser in die Messküvette 2 gepumpt werden. Mittels der genannten Dosierpumpe kann die Messküvette 2 entleert und die Luftblasen zum Mischen des Inhalts der Messküvette 2 erzeugt werden. Somit kann der Pipettierroboter 1 beispielsweise vier Pumpen umfassen. In anderen Ausführungsformen kann die Anzahl der Pumpen auch reduziert werden, beispielsweise wenn geeignete Verbindungen oder Ventile vorgesehen werden.

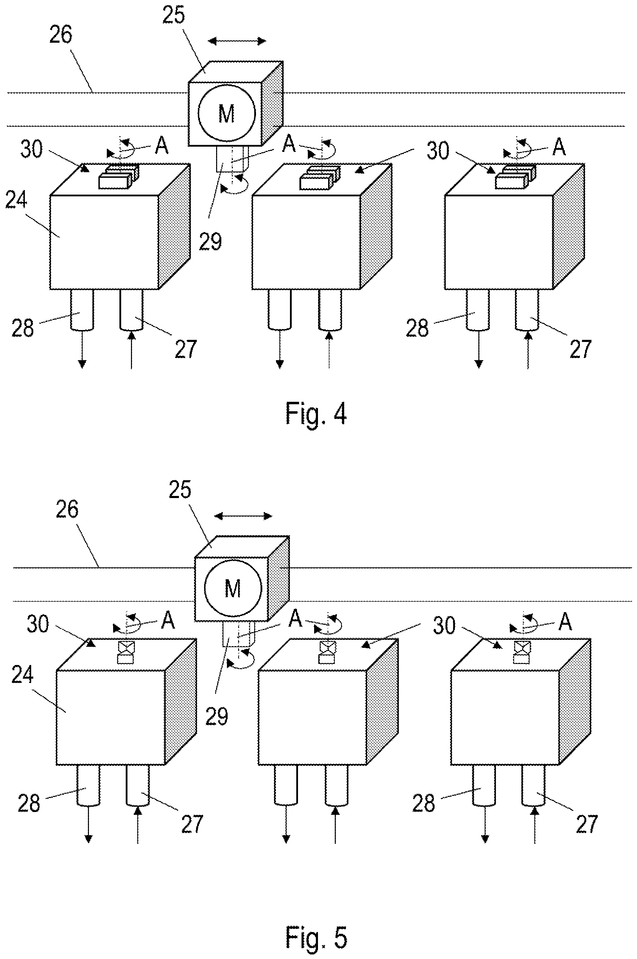

Alternativ zu der oben beschriebenen Ausführungsform, bei der die Pumpen Überdruck im Reagenzbehälter 3 erzeugen, können die Pumpen auch als Verdrängerpumpen ausgebildet werden, wie im Folgenden anhand der Figuren 4 und 5 beschrieben. Darin sind mehrere Pumpenköpfe 24 dargestellt, wobei jeweils ein Pumpenkopf 24 einem Reagenzbehälter 3 zugeordnet ist. Die Pumpenköpfe 24 könnten in einer Ausführungsform jeweils einen eigenen Pumpenmotor 25 aufweisen, in der dargestellten bevorzugten Ausführungsform teilen sich die mehreren Pumpenköpfe 24 jedoch jeweils einen gemeinsamen Pumpenmotor 25. Der Pumpenmotor 25, z.B. ein Schrittmotor, ist hierfür auf einer Linearführung 26 verschieblich gelagert, sodass er selektiv mit einem der Pumpenköpfe 24 verbunden werden kann.

Die Pumpenköpfe 24 weisen beispielsweise einen Schlauch mit einem Schlaucheingang 27 und einem Schlauchausgang 28 auf, wobei der Schlaucheingang 27 in den jeweiligen Reagenzbehälter 3 geführt ist. Weiters kann der Pumpenkopf 24 einen nicht weiter dargestellten Exzenter aufweisen, der auf den Schlauch einwirkt und dadurch Fluid vom Schlaucheingang 27 zum Schlauchausgang 28 fördert, wenn er durch den Pumpenmotor 25 um eine Rotationsachse A rotiert wird.

Um den Pumpenmotor 25 mit dem jeweiligen Pumpenkopf 24 bzw. dessen Exzenter zu koppeln, weist der Pumpenmotor 25 eine rotierbare Kopplungseinheit 29 auf, die beispielsweise einen rechteckigen oder nicht-kreisförmigen Querschnitt normal zur Rotationsachse A hat. Der Pumpenkopf 24 weist eine gegengleiche Aufnahme 30 auf, die in der Ausführungsform von Figur 4 durch zwei Quader gebildet ist, zwischen welche die Kopplungseinheit 29 des Pumpenmotor 25 eingeführt werden kann. Wird der Pumpenmotor 25 nun mittels der Führung 26 zum Pumpenkopf 24 bewegt, tritt die Kopplungseinheit 29 in die Aufnahme 30 ein. Wird die Kopplungseinheit 29 nun um die Rotationsachse A verdreht, dreht sich auch die Aufnahme 30 um die Rotationsachse A. So kann der Exzenter rotiert und Flüssigkeit gefördert werden.

Figur 5 zeigt, dass die Quader auch durch zwei oder mehr um die Rotationsachse A angeordnete extrudierte Dreiecke, d.h. dreiseitige Prismen, ersetzt werden können. Dadurch muss der Anstellwinkel zwischen der Kopplungseinheit 29 und der Aufnahme 30 weniger genau gewählt werden, um den Pumpenmotor 25 mit dem jeweiligen Pumpenkopf 24 zu koppeln.

Die Pumpenköpfe 24 können nicht nur für die Reagenzbehälter 3 vorgesehen werden, sondern auch, um Probewasser 5 oder Spülwasser in die Messküvette 2 einzubringen und/oder um weitere Flüssigkeiten zu dosieren. Weiters können auch zwei oder mehr Pumpenmotoren 25 auf unterschiedlichen Führungen 26 oder alternativ auf derselben Führung 26 eingesetzt werden, wobei die Pumpenmotoren 25 bevorzugt jeweils mit einem anderen Satz von Pumpenköpfen 24 koppelbar sind. Beispielsweise könnte der Pipettierroboter 1 zwanzig Pumpenköpfe 24 und zwei Pumpenmotoren 25 mit jeweils einer eigenen Führung 26 umfassen, wobei einer der Pumpenmotoren 25 mit zehn Pumpenköpfen 24 koppelbar ist und der andere der Pumpenmotoren 25 mit den zehn anderen Pumpenköpfe 24.

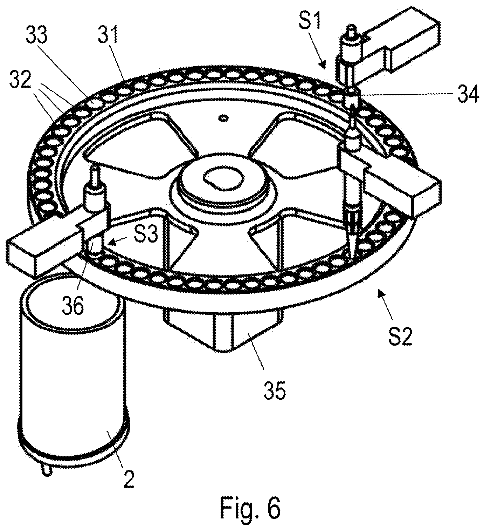

In Figur 6 ist ein weiterer Zusatz für den oben beschriebenen Pipettierroboter 1 dargestellt. In dieser Ausführungsform kann der Pipettierroboter 1 einen Kunststoffring 31, an dem mehrere Gefäße 32 ausgebildet sind, beispielsweise zumindest zwei, zumindest zehn oder zumindest fünfzig. In den Gefäßen 32 ist jeweils ein Pulver gelagert, mittels welchem ein für die vorbestimmte Eigenschaft repräsentativer Farbumschlag im Probewasser herbeiführbar ist. Üblicherweise wird das Pulver in Kombination mit den genannten flüssigen Reagenzien eingesetzt, um bestimmte Messwerte zu messen (z.B. die Calcium- oder Nitratkonzentration). Beispielsweise benötigt Calcium zwei flüssige Reagenzien und eine, die aus Pulver besteht.

Die Gefäße 32 sind auf einer ersten Seite, üblicherweise der Oberseite, mit einer Folie 33 verschlossen, welche beispielsweise aus Aluminium, Polyethylen oder Polyvinylalkohol besteht. Damit ist gewährleistet, dass das Pulver bis zur Verwendung trocken bleibt. Um das Pulver freizugeben, wird die Folie 33 mittels einer Nadel 34 eingestochen. Die Nadel 34 kann beispielsweise auf einem Magnetventil angeordnet sein, sodass die Stichbewegung der Nadel 34 durch das Magnetventil durchgeführt wird. In Figur 6 befindet sich die Nadel 34 an der Stelle S1, d.h. durch Ansteuern des Magnetventils wird die Folie 33 des Gefäßes 32 an der Stelle S1 eingestochen.

Der Kunststoffring 31 wird daraufhin durch einen Steppmotor 35 rotiert, sodass das Gefäß 32 mit eingestochener Folie 33 zu einer zweiten Stelle S2 bewegt wird, an welcher eine Flüssigkeit, beispielsweise Probewasser oder Osmosewasser, durch die eingestochene Folie 33 in das Gefäß 32 gefüllt wird. Das Pulver löst sich dadurch in dieser Flüssigkeit auf.

Danach wird das das Gefäß 32 mit eingestochener Folie 33 und gelöstem Pulver zu einer dritten Stelle S3 bewegt wird, um das gelöste Pulver in die Messküvette 2 zu verbringen. Die dritte Stelle S3 kann beispielsweise unmittelbar über der Messküvette 2 liegen oder über einer Rutsche oder einem Schlauch, die bzw. der das gelöste Pulver zur Messküvette 2 verbringt. Um das in der Flüssigkeit gelöste Pulver freizugeben, kann beispielsweise eine zweite Folie eingestochen werden, welche die Unterseite des Gefäßes 32 bildet. In diesem Fall umfasst das Gefäß 32 z.B. eine ringförmige Kunststoffwand, die einstückig am Kunststoffring 31 ausgebildet ist, und zwei Folien, welche die Oberseite und die Unterseite der ringförmigen Kunststoffwand verschließen. Die zweite Folie kann mittels einer weiteren Nadel 36 durchstochen werden, die beispielsweise von oben durch das Gefäß 32 stechen kann, um das in der Flüssigkeit gelöste Pulver freizugeben.

Alternativ könnte die zweite Folie wasserlöslich ausgebildet sein und z.B. aus wasserlöslichem Polyvinylalkohol (PVA) bestehen. Hierbei wird gewartet, bis die zweite Folie nach dem Einfüllen der Flüssigkeit von selbst bricht und das in der Flüssigkeit gelöste Pulver in die Messküvette 2 fällt. In diesem Fall fällt die zweite Stelle S2 bevorzugt mit der dritten Stelle S3 zusammen. Mittels der genannten Kamera 6 kann wiederum erkannt werden, wann die Flüssigkeit mit gelöstem Pulver in die Messküvette 2 eintropft, um mit dem nächsten Verfahrensschritten fortzufahren. Im Falle der wasserlöslichen Folie kann der Mischvorgang durch Bewegen des Kunststoffringes 31, beispielsweise mehrmaliges vor- und zurückdrehen mittels des Steppmotors, optional weiter angeregt werden. Die Bewegung auf der wasserlöslichen Folie hat denselben Effekt wie ein Rühren und führt dazu, dass sie sich schneller löst.

Die Ausführungsformen des Verbringens des gelösten Pulvers in die Messküvette 2 sind jedoch nicht ausschließlich auf den genannten verdrehbaren Kunststoffring beschränkt, sondern es könnte im Allgemeinen auch einfach ein Gefäß eingesetzt werden, das verkippt wird, um ein in einer Flüssigkeit gelöstes Pulver in die Messküvette zu schütten. Auch sind Alternativen zum Öffnen der Folie denkbar, beispielsweise ein Abziehen der Folie. Im Allgemeinen könnten die oben beschriebenen flüssigen Reagenzien dadurch hergestellt sein, dass zuvor solides Pulver mittels einer Flüssigkeit gelöst wird.

## Patentansprüche

1. Pipettierroboter (1) zur Bestimmung zumindest einer vorbestimmten Eigenschaft von Probewasser (5), insbesondere von Aquarienwasser, umfassend
- eine Messküvette (2) zur Aufnahme des Probewassers (5),
- zumindest einen Reagenzbehälter (3) zur Aufnahme eines flüssigen Reagenzes (10), mittels welches Reagenzes (10) ein für die vorbestimmte Eigenschaft repräsentativer Farbumschlag im Probewasser (5) herbeiführbar ist,
- eine Pumpe (4) zum Verbringen des Reagenzes (10) vom Reagenzbehälter (3) in die Messküvette (2) über einen Reagenzeinlass (12) an einer Oberseite der Messküvette (2), wodurch das Reagenz (10) in die Messküvette (2) eintropfbar ist, und
- eine Kamera (6) zur Aufnahme von Bildern der Messküvette (2) und des in der Messküvette (2) befindlichen Probewassers (5),
- einen Hintergrund (7), welcher auf der der Kamera (6) abgewandten Seite der Messküvette (2) angeordnet ist und im Blickfeld der Kamera (6) liegt, wobei auf dem Hintergrund (7) zur Kontrastgebung Flächen (16) in zumindest zwei unterschiedlichen Farben in einem vorbestimmten Muster abgebildet sind, wobei das Muster bevorzugt ein Schachbrettmuster mit schwarzen und weißen Flächen (16) ist, und wobei auf dem Hintergrund (7) zur Farbreferenzgebung zumindest drei Referenzfarbpunkte (17) abgebildet sind, von denen zumindest einer Rot, einer Grün und einer Blau ist, und
- eine Auswerteeinheit (8), die dazu ausgebildet ist, anhand der von der Kamera (6) aufgezeichneten Bilder die Füllmenge des Probewassers (5) in der Messküvette (2) zu bestimmen, die Anzahl von in die Messküvette (2) eingetropften Reagenztröpfchen (15) zu zählen und aus der gezählten Anzahl an Reagenztröpfchen (15) in Kombination mit dem aus den Bildern ermittelten Farbumschlag des Probewassers (5) die zumindest eine vorbestimmte Eigenschaft des Probewassers (5) zu bestimmen.

2. Pipettierroboter (1) nach Anspruch 1, wobei der Reagenzeinlass (12) dazu ausgebildet ist, Reagenztröpfchen (15) mit einer vorbestimmten Tröpfchengröße auszugeben, und wobei die genannten Flächen (16) eine durchschnittliche Höhe und/oder eine durchschnittliche Länge haben, die maximal der Hälfte der Tröpfchengröße entspricht.

3. Pipettierroboter (1) nach Anspruch 1 oder 2, wobei die Pumpe (4) zumindest einen motorlosen Pumpenkopf (24) und einen mit dem motorlosen Pumpenkopf (24) koppelbaren Pumpenmotor (25) umfasst, wobei der Pipettierroboter (1) bevorzugt zumindest zwei Reagenzbehälter (3) und einen eigenen motorlosen Pumpenkopf (24) für jeden der zumindest zwei Reagenzbehälter (3) umfasst, und wobei der Pumpenmotor (25) auf einer Führung (26) gelagert ist und durch Verschieben auf der Führung (26) selektiv mit den Pumpenköpfen (24) der zumindest zwei Reagenzbehälter (3) koppelbar ist, wobei der Pumpenmotor (25) bevorzugt eine um eine Rotationsachse (A) rotierbare Kopplungseinheit (29) umfasst, welche zur Kopplung mit dem motorlosen Pumpenkopf (24) in eine gegengleiche Aufnahme (30) des motorlosen Pumpenkopfs (24) einführbar ist, wobei die Aufnahme (30) des Pumpenkopfs (24) bevorzugt zumindest zwei symmetrisch um eine Rotationsachse (A) angeordnete Stifte aufweist, die im Querschnitt die Form eines Dreiecks oder Kreissektors haben.

4. Pipettierroboter (1) nach einem der Ansprüche 1 bis 3, wobei der Pipettierroboter (1) zumindest zwei, bevorzugt zumindest zehn, Reagenzbehälter (3) aufweist, die jeweils mit der Pumpe (4) und der Messküvette (2) in Verbindung stehen, wobei die Reagenzbehälter (3) jeweils ein ansteuerbares Ventil, bevorzugt ein Magnetventil (14), zur selektiven Verbindung zur Pumpe (4) und/oder zur Messküvette (2) umfassen, und/oder wobei der bzw. die Reagenzbehälter (3) auswechselbar im Pipettierroboter (1) vorgesehen sind und bevorzugt hängend in diesen eingebracht werden können.

5. Pipettierroboter (1) nach einem der Ansprüche 1 bis 4, wobei der Pipettierroboter (1) mit einer Quelle des Probewassers verbunden und dazu ausgebildet ist, so lange Probewasser (5) in die Messküvette (2) zu pumpen, bis die Auswerteeinheit (8) anhand der von der Kamera (6) aufgenommenen Bilder eine gewünschte Füllmenge in der Messküvette (2) erkennt, wobei die Auswerteeinheit (8) bevorzugt dazu ausgebildet ist, das Erreichen der gewünschten Füllmenge durch Abgleich mit einer in der Auswerteeinheit (8) hinterlegten Referenzposition in den von der Kamera (6) aufgenommenen Bildern zu bestimmen, wobei die Messküvette (2) besonders bevorzugt einen transparenten Probewassereinlass (9) zum Zuführen des Probewassers (5) aufweist und der Pipettierroboter (1) dazu ausgebildet ist, einen Pumpvorgang des Probewassers (5) in die Messküvette (2) so lange mit einer ersten Probewasserpumpgeschwindigkeit durchzuführen, bis die Auswerteeinheit (8) das Vorhandensein des Probewassers (5) im Probewassereinlass (12) detektiert, und den Pumpvorgang so lange mit einer zweiten Probewasserpumpgeschwindigkeit durchzuführen, die geringer ist als die erste Probewasserpumpgeschwindigkeit, bis die Auswerteeinheit (8) Probewasser (5) an der Referenzposition erkennt.

6. Pipettierroboter (1) nach einem der Ansprüche 1 bis 5, wobei der Reagenzeinlass (12) transparent ist und wobei die Pumpe (4) dazu ausgebildet ist, einen Pumpvorgang des Reagenzes (10) so lange mit einer ersten Reagenzpumpgeschwindigkeit durchzuführen, bis die Auswerteeinheit (8) das Vorhandensein des Reagenzes im Reagenzeinlass detektiert, und den Pumpvorgang so lange mit einer zweiten Reagenzpumpgeschwindigkeit durchzuführen, die geringer ist als die erste Reagenzpumpgeschwindigkeit, bis die Auswerteeinheit (8) eine vorbestimmte Anzahl von Reagenztröpfchen (15) gezählt oder einen Farbumschlag im Probewasser (5) detektiert hat.

7. Pipettierroboter (1) nach einem der Ansprüche 1 bis 6, wobei die Auswerteeinheit (8) dazu ausgebildet ist, die vom Reagenzeinlass (12) in die Messküvette (2) tropfenden Reagenztröpfchen (15) zu zählen und die Pumpe (4) abzuschalten, sobald eine vorbestimmte Anzahl von Reagenztröpfchen (15) oder ein vorbestimmter Farbumschlag des Probewassers (5) erkannt wurde.

8. Pipettierroboter (1) nach einem der Ansprüche 1 bis 7, wobei der Hintergrund (7) oder die Messküvette (2) einen vertikalen Streifen (18), bevorzugt einen vertikalen roten Streifen, zur Ermittlung eines Salzgehaltes aufweist, wobei die Messküvette (2) weiters bevorzugt eine Lupe (19) aufweist und die Kamera (6), die Lupe (19) und der vertikale Streifen (18) derart positioniert sind, dass der vertikale Streifen (18) auf den von der Kamera (6) aufgenommenen Bildern bei einem Salzgehalt von 3,5% im Probewasser (5) vergrößert dargestellt ist, und/oder wobei der Hintergrund (7) oder die Messküvette (2) zumindest einen horizontalen Streifen (20), bevorzugt zumindest einen horizontalen grünen Streifen, zur Plausibilisierung einer Füllmenge aufweist.

9. Pipettierroboter (1) nach einem der Ansprüche 1 bis 8, wobei die Messküvette (2) an der Unterseite eine Auslassöffnung (21) zum Entleeren der Messküvette (2) aufweist, wobei der Pipettierroboter (1) bevorzugt dazu ausgebildet ist, zum Mischen des Probewassers (5) und des eingetropften Reagenzes (10) zumindest eine Luftblase über die genannte Auslassöffnung (21) einströmen zu lassen.

10. Pipettierroboter (1) nach einem der Ansprüche 1 bis 9, wobei die vorbestimmte Eigenschaft ein pH-Wert ist, wobei das Reagenz (10) derart gewählt ist, einen Farbumschlag des Probewassers (5) in Abhängigkeit des pH-Wertes zu bewirken, wobei die Auswerteeinheit (8) dazu ausgebildet ist, in Abhängigkeit des Farbumschlages den pH-Wert des Probewassers (5) zu bestimmen, wobei weiters eine Sonde zur Messung des pH-Wertes mit dem Pipettierroboter (1) verbunden ist und die Auswerteeinheit (8) dazu ausgebildet ist, die Sonde mittels des pH-Wertes zu kalibrieren, der von der Auswerteeinheit (8) über den Farbumschlag bestimmt wurde.

11. Pipettierroboter (1) nach einem der Ansprüche 1 bis 10, wobei der Pipettierroboter (1) eine lichtundurchlässige Kammer umfasst, in welcher sich die Messküvette (2) befindet, wobei zumindest eine Leuchte (23), bevorzugt eine LED-Leuchte, zur Ausleuchtung der Messküvette (2) in der Kammer angeordnet ist, und wobei sich die Leuchte (23) bevorzugt neben der Messküvette (2) befindet.

12. Pipettierroboter (1) nach einem der Ansprüche 1 bis 11, umfassend einen Beschleunigungsmesssensor und/oder einen Neigungssensor, wobei die Auswerteeinheit (8) dazu ausgebildet ist, eine Messung der vorbestimmten Eigenschaft zu invalideren, wenn der Beschleunigungsmesssensor und/oder der Neigungssensor eine Schieflage oder einen Stoß des Pipettierroboters (1) anzeigt.

13. Pipettierroboter (1) nach einem der Ansprüche 1 bis 12, ferner umfassend ein Gefäß (32), in welchem ein Pulver gelagert ist, mittels welchem Pulver ein für die vorbestimmte Eigenschaft repräsentativer Farbumschlag im Probewasser (5) herbeiführbar ist, wobei das Gefäß (32) zumindest auf einer ersten Seite, bevorzugt auf der Oberseite, mittels einer ersten Folie (33) verschlossen ist, wobei der Pipettierroboter (1) ferner eine Nadel (34) umfasst und dazu ausgebildet ist, die erste Folie (33) mittels der Nadel (34) einzustechen, eine Flüssigkeit zur Lösung des Pulvers in das Gefäß (32) zu füllen und das in der Flüssigkeit gelöste Pulver in die Messküvette (2) zu überführen, wobei das Gefäß (32) bevorzugt auch an einer zweiten Seite, bevorzugt auf der Unterseite, mittels einer zweiten Folie verschlossen ist, durch welche das in der Flüssigkeit gelöste Pulver in die Messküvette (2) überführbar ist wobei der Pipettierroboter (1) bevorzugt einen optional modular in den Pipettierroboter (1) einsetzbaren Kunststoffring (31) und zumindest zwei der genannten Gefäße (32) umfasst, in denen jeweils Pulver gelagert ist, wobei die Gefäße (32) um den Umfang des Kunststoffrings (31) verteilt angeordnet sind und der Pipettierroboter (1) einen Steppmotor (35) zum Drehen des Kunststoffringes (31) umfasst, mittels welchem die Gefäße selektiv zur Nadel (34), zur Zuführstelle der Flüssigkeit und/oder zur Überführungsstelle zur Messküvette (2) verdrehbar sind.

14. Verwendung des Pipettierroboters (1) nach einem der Ansprüche 1 bis 13 zur Bestimmung einer vorbestimmten Eigenschaft von Probewasser (5), wobei das Probewasser (5) Aquarienwasser ist und die vorbestimmte Eigenschaft bevorzugt eine Alkalinität, eine Calciumhärte, eine Magnesiumhärte, ein Phosphatwert, ein Nitratwert und/oder ein pH-Wert ist.

15. Verfahren zum Bestimmen einer vorbestimmten Eigenschaft von Probewasser (5) mittels eines Pipettierroboters (1) nach einem der Ansprüche 1 bis 13, umfassend die Schritte:
- Einfüllen von Probewasser (5) in die Messküvette (2),
- Verbringen des Reagenzes (10) vom Reagenzbehälter (3) in die Messküvette (3) mittels der Pumpe (4),
- Aufnehmen von Bildern der Messküvette (2) durch die Kamera (6),
- Zählen von Reagenztröpfchen (15), die über den Reagenzeinlass (12) in die Messküvette (2) eingetropft werden, und Ermitteln eines Farbumschlages des Probewassers (5) durch die Auswerteeinheit (8) anhand der von der Kamera (6) aufgenommenen Bilder, und
- Bestimmen der vorbestimmten Eigenschaft anhand der gezählten Reagenztröpfchen (15) und/oder des ermittelten Farbumschlages.

## Claims

1. Pipetting robot (1) for determining at least one predetermined property of sample water (5), in particular aquarium water, comprising
- a measuring cuvette (2) for holding the sample water (5),
- at least one reagent container (3) for holding a liquid reagent (10), by means of which reagent (10) a color change representative of the predetermined property can be brought about in the sample water (5),
- a pump (4) for transferring the reagent (10) from the reagent container (3) into the measuring cuvette (2) via a reagent inlet (12) at an upper side of the measuring cuvette (2), whereby the reagent (10) can be dripped into the measuring cuvette (2), and
- a camera (6) for recording images of the measuring cuvette (2) and the sample water (5) in the measuring cuvette (2),
- a background (7) which is arranged on the side of the measuring cuvette (2) facing away from the camera (6) and lies in the field of view of the camera (6), wherein areas (16) in at least two different colors are depicted on the background (7) in a predetermined pattern for providing contrast, wherein the pattern is preferably a checkerboard pattern with black and white areas (16), and wherein at least three reference color points (17) are depicted on the background (7) for providing color reference, of which at least one is red, one is green and one is blue, and
- an evaluation unit (8) which is designed to determine the filling quantity of the sample water (5) in the measuring cuvette (2) on the basis of the images recorded by the camera (6), to count the number of reagent droplets (15) dripping into the measuring cuvette (2) and to determine the at least one predetermined property of the sample water (5) from the counted number of reagent droplets (15) in combination with the color change of the sample water (5) determined from the images.

2. The pipetting robot (1) according to claim 1, wherein the reagent inlet (12) is designed to dispense reagent droplets (15) having a predetermined droplet size, and wherein said surfaces (16) have an average height and/or an average length corresponding to at most half the droplet size.

3. The pipetting robot (1) according to claim 1 or 2, wherein the pump (4) comprises at least one motorless pump head (24) and a pump motor (25) which can be coupled to the motorless pump head (24), wherein the pipetting robot (1) preferably comprises at least two reagent containers (3) and a separate motorless pump head (24) for each of the at least two reagent containers (3), and wherein the pump motor (25) is mounted on a guide (26) and can be selectively coupled to the pump heads (24) of the at least two reagent containers (3) by displacement on the guide (26), wherein the pump motor (25) preferably comprises a coupling unit (29) which is rotatable about an axis of rotation (A) and which can be introduced into an opposing receptacle (30) of the motorless pump head (24) for coupling with the motorless pump head (24), wherein the receptacle (30) of the pump head (24) preferably has at least two pins which are arranged symmetrically about an axis of rotation (A) and which have the shape of a triangle or sector of a circle in cross-section.

4. The pipetting robot (1) according to any one of claims 1 to 3, wherein the pipetting robot (1) has at least two, preferably at least ten, reagent containers (3), which are each connected to the pump (4) and the measuring cuvette (2), wherein the reagent containers (3) each comprise a controllable valve, preferably a solenoid valve (14), for selective connection to the pump (4) and/or to the measuring cuvette (2), and/or wherein the reagent container(s) (3) is/are provided in the pipetting robot (1) so as to be exchangeable and can preferably be inserted into the latter in a suspended manner.

5. The pipetting robot (1) according to any one of claims 1 to 4, wherein the pipetting robot (1) is connected to a source of the sample water and is designed to pump sample water (5) into the measuring cuvette (2) until the evaluation unit (8) recognizes a desired filling quantity in the measuring cuvette (2) on the basis of the images recorded by the camera (6), wherein the evaluation unit (8) is preferably designed to determine whether the desired filling quantity has been reached by comparing a reference position stored in the evaluation unit (8) in the images recorded by the camera (6), wherein the measuring cuvette (2) particularly preferably has a transparent sample water inlet (9) for supplying the sample water (5) and the pipetting robot (1) is designed to carry out a pumping process of the sample water (5) into the measuring cuvette (2) at a first sample water pumping speed until the evaluation unit (8) is able to recognize the sample water (5) in the measuring cuvette (2), until the evaluation unit (8) detects the presence of the sample water (5) in the sample water inlet (12), and to carry out the pumping process at a second sample water pumping speed which is lower than the first sample water pumping speed until the evaluation unit (8) recognizes sample water (5) at the reference position.

6. The pipetting robot (1) according to any one of claims 1 to 5, wherein the reagent inlet (12) is transparent and wherein the pump (4) is designed to carry out a pumping operation of the reagent (10) at a first reagent pumping speed until the evaluation unit (8) detects the presence of the reagent in the reagent inlet, and to carry out the pumping process at a second reagent pumping speed which is lower than the first reagent pumping speed until the evaluation unit (8) has counted a predetermined number of reagent droplets (15) or has detected a color change in the sample water (5).

7. The pipetting robot (1) according to any one of claims 1 to 6, wherein the evaluation unit (8) is designed to count the reagent droplets (15) dripping from the reagent inlet (12) into the measuring cuvette (2) and to switch off the pump (4) as soon as a predetermined number of reagent droplets (15) or a predetermined color change of the sample water (5) has been detected.

8. The pipetting robot (1) according to any one of claims 1 to 7, wherein the background (7) or the measuring cuvette (2) has a vertical strip (18), preferably a vertical red strip, for determining a salt content, wherein the measuring cuvette (2) further preferably has a magnifying glass (19) and the camera (6), the magnifying glass (19) and the vertical strip (18) are positioned in such a way that the vertical strip (18) is shown enlarged on the images recorded by the camera (6) at a salt content of 3.5% in the sample water (5), and/or wherein the background (7) or the measuring cuvette (2) has at least one horizontal strip (20), preferably at least one horizontal green strip, for plausibilisation of a filling quantity.

9. The pipetting robot (1) according to any one of claims 1 to 8, wherein the measuring cuvette (2) has an outlet opening (21) on the underside for emptying the measuring cuvette (2), wherein the pipetting robot (1) is preferably designed to allow at least one air bubble to flow in via said outlet opening (21) for mixing the sample water (5) and the dripped reagent (10).

10. The pipetting robot (1) according to any one of claims 1 to 9, wherein the predetermined property is a pH value, wherein the reagent (10) is selected such that it causes a color change in the sample water (5) as a function of the pH value, wherein the evaluation unit (8) is designed to determine the pH value of the sample water (5) as a function of the color change, wherein furthermore a probe for measuring the pH value is connected to the pipetting robot (1) and the evaluation unit (8) is designed to calibrate the probe by means of the pH value which was determined by the evaluation unit (8) via the color change.

11. The pipetting robot (1) according to any one of claims 1 to 10, wherein the pipetting robot (1) comprises an opaque chamber in which the measuring cuvette (2) is located, wherein at least one light (23), preferably an LED light, is arranged in the chamber for illuminating the measuring cuvette (2), and wherein the light (23) is preferably located next to the measuring cuvette (2).

12. The pipetting robot (1) according to any one of claims 1 to 11, comprising an acceleration measurement sensor and/or an inclination sensor, wherein the evaluation unit (8) is designed to invalidate a measurement of the predetermined property when the acceleration measurement sensor and/or the inclination sensor indicates an inclination or an impact of the pipetting robot (1).

13. The pipetting robot (1) according to any one of claims 1 to 12, further comprising a vessel (32) in which a powder is stored, by means of which powder a color change representative of the predetermined property can be brought about in the sample water (5), wherein the vessel (32) is closed at least on a first side, preferably on the upper side, by means of a first foil (33), wherein the pipetting robot (1) further comprises a needle (34) and is designed to pierce the first foil (33) by means of the needle (34), to fill a liquid for dissolving the powder into the vessel (32) and to transfer the powder dissolved in the liquid into the measuring cuvette (2), wherein the vessel (32) is preferably also closed on a second side, preferably on the underside, by means of a needle (34), preferably on the underside, by means of a second film, through which the powder dissolved in the liquid can be transferred into the measuring cuvette (2), the pipetting robot (1) preferably comprising a plastic ring (31), which can optionally be inserted into the pipetting robot (1) in a modular manner, and at least two of the said vessels (32), in each of which powder is stored, wherein the vessels (32) are arranged distributed around the circumference of the plastic ring (31) and the pipetting robot (1) comprises a stepper motor (35) for rotating the plastic ring (31), by means of which the vessels can be rotated selectively towards the needle (34), towards the liquid feed point and/or towards the transfer point to the measuring cuvette (2).

14. Use of the pipetting robot (1) according to any one of claims 1 to 13 for determining a predetermined property of sample water (5), wherein the sample water (5) is aquarium water and the predetermined property is preferably an alkalinity, a calcium hardness, a magnesium hardness, a phosphate value, a nitrate value and/or a pH value.

15. A method for determining a predetermined property of sample water (5) by means of a pipetting robot (1) according to any one of claims 1 to 13, comprising the steps of:
- Filling sample water (5) into the measuring cuvette (2),
- Transferring the reagent (10) from the reagent container (3) to the measuring cell (3) using the pump (4),
- Recording images of the measuring cuvette (2) by the camera (6),
- Counting of reagent droplets (15) which are dropped into the measuring cuvette (2) via the reagent inlet (12), and determination of a color change of the sample water (5) by the evaluation unit (8) on the basis of the images recorded by the camera (6), and
- Determining the predetermined property on the basis of the counted reagent droplets (15) and/or the determined color change.

## Revendications

1. Robot de pipetage (1) pour la détermination d'au moins une propriété prédéterminée d'eau d'échantillon (5), en particulier d'eau d'aquarium, comprenant
- une cuvette de mesure (2) destinée à recevoir l'eau d'échantillonnage (5),
- au moins un récipient de réactif (3) pour recevoir un réactif liquide (10), au moyen duquel réactif (10) un changement de couleur représentatif de la propriété prédéterminée peut être provoqué dans l'eau d'échantillon (5),
- une pompe (4) pour transférer le réactif (10) du réservoir de réactif (3) dans la cuvette de mesure (2) via une entrée de réactif (12) sur un côté supérieur de la cuvette de mesure (2), permettant ainsi au réactif (10) de s'égoutter dans la cuvette de mesure (2), et
- une caméra (6) pour enregistrer des images de la cuvette de mesure (2) et de l'eau d'échantillon (5) se trouvant dans la cuvette de mesure (2),
- un fond (7) qui est disposé sur le côté de la cuvette de mesure (2) opposé à la caméra (6) et qui se trouve dans le champ de vision de la caméra (6), des surfaces (16) d'au moins deux couleurs différentes étant représentées sur le fond (7) pour la création de contraste dans un modèle prédéterminé, le modèle étant de préférence un modèle en damier avec des surfaces noires et blanches (16), et au moins trois points de couleur de référence (17) étant représentés sur le fond (7) pour la création de référence de couleur, dont au moins un est rouge, un vert et un bleu, et
- une unité d'évaluation (8) qui est conçue pour déterminer, à l'aide des images enregistrées par la caméra (6), la quantité de remplissage de l'eau d'échantillon (5) dans la cuvette de mesure (2), pour compter le nombre de gouttelettes de réactif (15) tombées dans la cuvette de mesure (2) et pour déterminer, à partir du nombre compté de gouttelettes de réactif (15) en combinaison avec le changement de couleur de l'eau d'échantillon (5) déterminé à partir des images, l'au moins une propriété prédéterminée de l'eau d'échantillon (5).

2. Robot de pipetage (1) selon la revendication 1, dans lequel l'entrée de réactif (12) est adaptée pour distribuer des gouttelettes de réactif (15) ayant une taille de gouttelette prédéterminée, et dans lequel lesdites surfaces (16) ont une hauteur moyenne et/ou une longueur moyenne qui correspond au maximum à la moitié de la taille de gouttelette.

3. Robot de pipetage (1) selon la revendication 1 ou 2, dans lequel la pompe (4) comprend au moins une tête de pompe sans moteur (24) et un moteur de pompe (25) pouvant être couplé à la tête de pompe sans moteur (24), dans lequel le robot de pipetage (1) comprend de préférence au moins deux récipients de réactifs (3) et une tête de pompe sans moteur (24) propre pour chacun des au moins deux récipients de réactifs (3), et le moteur de pompe (25) étant monté sur un guide (26) et pouvant être couplé sélectivement aux têtes de pompe (24) des au moins deux récipients à réactifs (3) par déplacement sur le guide (26), le moteur de pompe (25) comprenant de préférence une unité de couplage (29) pouvant tourner autour d'un axe de rotation (A), qui peut être introduite dans un logement (30) opposé de la tête de pompe (24) sans moteur pour le couplage avec la tête de pompe (24) sans moteur, le logement (30) de la tête de pompe (24) présentant de préférence au moins deux broches disposées symétriquement autour d'un axe de rotation (A), qui ont en section transversale la forme d'un triangle ou d'un secteur circulaire.

4. Robot de pipetage (1) selon l'une des revendications 1 à 3, dans lequel le robot de pipetage (1) présente au moins deux, de préférence au moins dix, récipients de réactifs (3) qui sont respectivement en liaison avec la pompe (4) et la cuvette de mesure (2), dans lequel les récipients de réactifs (3) comprennent respectivement une vanne commandable, de préférence une électrovanne (14), pour la liaison sélective à la pompe (4) et/ou à la cuvette de mesure (2), et/ou dans lequel le ou les récipients de réactifs (3) peuvent être montés de manière amovible dans le robot de pipetage (1).

5. Robot de pipetage (1) selon l'une des revendications 1 à 4, dans lequel le robot de pipetage (1) est relié à une source de l'eau d'échantillon et est conçu pour pomper de l'eau d'échantillon (5) dans la cuvette de mesure (2) jusqu'à ce que l'unité d'évaluation (8) reconnaisse une quantité de remplissage souhaitée dans la cuvette de mesure (2) à l'aide des images enregistrées par la caméra (6), l'unité d'évaluation (8) étant de préférence conçue pour déterminer l'obtention de la quantité de remplissage souhaitée par comparaison avec une position de référence enregistrée dans l'unité d'évaluation (8) dans les images prises par la caméra (6), la cuvette de mesure (2) présentant de manière particulièrement préférée une entrée d'eau d'échantillon transparente (9) pour l'amenée de l'eau d'échantillon (5) et le robot de pipetage (1) étant conçu pour effectuer un processus de pompage de l'eau d'échantillon (5) dans la cuvette de mesure (2) à une première vitesse de pompage d'eau d'échantillon aussi longtemps, jusqu'à ce que l'unité d'évaluation (8) détecte la présence de l'eau d'échantillon (5) dans l'entrée d'eau d'échantillon (12), et à effectuer le processus de pompage à une deuxième vitesse de pompage d'eau d'échantillon qui est inférieure à la première vitesse de pompage d'eau d'échantillon jusqu'à ce que l'unité d'évaluation (8) détecte de l'eau d'échantillon (5) à la position de référence.

6. Robot de pipetage (1) selon l'une des revendications 1 à 5, dans lequel l'entrée de réactif (12) est transparente et dans lequel la pompe (4) est configurée pour effectuer une opération de pompage du réactif (10) à une première vitesse de pompage du réactif jusqu'à ce que l'unité d'évaluation (8) détecte la présence du réactif dans l'entrée de réactif, et à effectuer le processus de pompage à une deuxième vitesse de pompage du réactif, qui est inférieure à la première vitesse de pompage du réactif, jusqu'à ce que l'unité d'évaluation (8) ait compté un nombre prédéterminé de gouttelettes de réactif (15) ou détecté un changement de couleur dans l'eau d'échantillonnage (5).

7. Robot de pipetage (1) selon l'une des revendications 1 à 6, dans lequel l'unité d'évaluation (8) est conçue pour compter les gouttelettes de réactif (15) s'égouttant de l'entrée de réactif (12) dans la cuvette de mesure (2) et pour arrêter la pompe (4) dès qu'un nombre prédéterminé de gouttelettes de réactif (15) ou un changement de couleur prédéterminé de l'eau d'échantillonnage (5) a été détecté.

8. Robot de pipetage (1) selon l'une des revendications 1 à 7, dans lequel le fond (7) ou la cuvette de mesure (2) présente une bande verticale (18), de préférence une bande rouge verticale, pour déterminer une teneur en sel, dans lequel la cuvette de mesure (2) présente en outre de préférence une loupe (19) et la caméra (6), la loupe (19) et la bande verticale (18) sont positionnées de telle sorte, que la bande verticale (18) est représentée agrandie sur les images prises par la caméra (6) pour une teneur en sel de 3,5% dans l'eau d'échantillonnage (5), et/ou dans lequel le fond (7) ou la cuvette de mesure (2) présente au moins une bande horizontale (20), de préférence au moins une bande verte horizontale, pour la plausibilisation d'une quantité de remplissage.

9. Robot de pipetage (1) selon l'une quelconque des revendications 1 à 8, dans lequel la cuvette de mesure (2) présente sur sa face inférieure une ouverture de sortie (21) pour vider la cuvette de mesure (2), le robot de pipetage (1) étant de préférence conçu pour laisser entrer au moins une bulle d'air par ladite ouverture de sortie (21) pour mélanger l'eau d'échantillon (5) et le réactif (10) qui a été égoutté.

10. Robot de pipetage (1) selon l'une des revendications 1 à 9, dans lequel la propriété prédéterminée est une valeur de pH, le réactif (10) étant choisi de manière à provoquer un changement de couleur de l'eau d'échantillon (5) en fonction de la valeur de pH, l'unité d'évaluation (8) étant conçue pour cela, déterminer la valeur du pH de l'eau d'échantillon (5) en fonction du changement de couleur, une sonde de mesure de la valeur du pH étant en outre reliée au robot de pipetage (1) et l'unité d'évaluation (8) étant conçue pour calibrer la sonde au moyen de la valeur du pH qui a été déterminée par l'unité d'évaluation (8) par l'intermédiaire du changement de couleur.

11. Robot de pipetage (1) selon l'une des revendications 1 à 10, dans lequel le robot de pipetage (1) comprend une chambre opaque dans laquelle se trouve la cuvette de mesure (2), dans lequel au moins une lampe (23), de préférence une lampe LED, est disposée dans la chambre pour éclairer la cuvette de mesure (2), et dans lequel la lampe (23) se trouve de préférence à côté de la cuvette de mesure (2).

12. Robot de pipetage (1) selon l'une des revendications 1 à 11, comprenant un capteur d'accélération et/ou un capteur d'inclinaison, l'unité d'évaluation (8) étant conçue pour invalider une mesure de la propriété prédéterminée lorsque le capteur d'accélération et/ou le capteur d'inclinaison indiquent une inclinaison ou un choc du robot de pipetage (1).

13. Robot de pipetage (1) selon l'une des revendications 1 à 12, comprenant en outre un récipient (32) dans lequel est stockée une poudre, au moyen de laquelle poudre un changement de couleur représentatif de la propriété prédéterminée peut être provoqué dans l'eau échantillon (5), le récipient (32) étant fermé au moins sur un premier côté, de préférence sur le côté supérieur, au moyen d'un premier film (33), le robot de pipetage (1) comprenant en outre une aiguille (34) et étant conçu pour percer le premier film (33) au moyen de l'aiguille (34), pour remplir le récipient (32) d'un liquide destiné à dissoudre la poudre et pour transférer la poudre dissoute dans le liquide dans la cuvette de mesure (2), le récipient (32) étant de préférence également fermé sur un deuxième côté, de préférence sur le côté inférieur, au moyen d'un deuxième film à travers lequel la poudre dissoute dans le liquide peut être transférée dans la cuvette de mesure (2), le robot de pipetage (1) comprenant de préférence un anneau en plastique (31) pouvant être inséré de manière modulaire en option dans le robot de pipetage (1) et au moins deux des récipients (32) mentionnés, dans lesquels de la poudre est respectivement stockée, les récipients (32) étant répartis autour de la périphérie de l'anneau en plastique (31) et le robot de pipetage (1) comprenant un moteur pas à pas (35) pour faire tourner l'anneau en plastique (31), au moyen duquel les récipients peuvent être tournés sélectivement vers l'aiguille (34), vers le point d'amenée du liquide et/ou vers le point de transfert vers la cuvette de mesure (2).

14. Utilisation du robot de pipetage (1) selon l'une des revendications 1 à 13 pour déterminer une propriété prédéterminée de l'eau d'échantillon (5), l'eau d'échantillon (5) étant de l'eau d'aquarium et la propriété prédéterminée étant de préférence une alcalinité, une dureté du calcium, une dureté du magnésium, une valeur de phosphate, une valeur de nitrate et/ou une valeur de pH.

15. Procédé de détermination d'une propriété prédéterminée d'une eau d'échantillon (5) au moyen d'un robot de pipetage (1) selon l'une quelconque des revendications 1 à 13, comprenant les étapes consistant à :
- Remplir l'eau d'échantillon (5) dans la cuvette de mesure (2),
- Transférer le réactif (10) du récipient de réactif (3) dans la cuvette de mesure (3) au moyen de la pompe (4),
- Prendre d'images de la cuvette de mesure (2) par la caméra (6),
- Compter des gouttelettes de réactif (15) qui sont introduites goutte à goutte dans la cuvette de mesure (2) via l'entrée de réactif (12), et déterminer un changement de couleur de l'eau d'échantillon (5) par l'unité d'évaluation (8) à l'aide des images enregistrées par la caméra (6), et
- Déterminer la propriété prédéterminée à l'aide des gouttelettes de réactif (15) comptées et/ou du changement de couleur déterminé.
